(19) Europäisches Patentamt
European Patent Office
Office européen des brevets

(11) EP 1 302 475 A1

(12) EUROPEAN PATENT APPLICATION
published in accordance with Art. 158(3) EPC

(43) Date of publication:
**16.04.2003 Bulletin 2003/16**

(21) Application number: **01945757.1**

(22) Date of filing: **02.07.2001**

(51) Int Cl.7: **C07H 23/00**, A61K 31/702, A61P 31/04

(86) International application number:
**PCT/JP01/05717**

(87) International publication number:
**WO 02/002588 (10.01.2002 Gazette 2002/02)**

(84) Designated Contracting States:
**AT BE CH CY DE DK ES FI FR GB GR IE IT LI LU MC NL PT SE TR**

(30) Priority: **30.06.2000 JP 2000200139**
**15.03.2001 JP 2001075081**

(71) Applicant: **JAPAN SCIENCE AND TECHNOLOGY CORPORATION**
**Kawaguchi-shi, Saitama 332-0012 (JP)**

(72) Inventors:
- **MATSUOKA, Koji**
  **Hasuda-shi, Saitama 349-0114 (JP)**
- **TERUNUMA, Taiyo**
  **Saitama-shi, Saitama 331-0044 (JP)**
- **KUZUHARA, Hiromi**
  **Tokorazawa-shi, Saitama 359-0025 (JP)**
- **SUZUKI, Yasuo**
  **Shizuoka-shi, Shizuoka 420-0911 (JP)**
- **NATORI, Yasuhiro**
  **Shinjyuku-ku, Tokyo 169-0072 (JP)**
- **NISHIKAWA, Kiyotaka**
  **Bunkyo-ku, Tokyo 113-0024 (JP)**

(74) Representative: **Calamita, Roberto**
**Frank B. Dehn & Co.,**
**European Patent Attorneys,**
**179 Queen Victoria Street**
**London EC4V 4EL (GB)**

(54) **SUGAR CHAIN-CONTAINING CARBOSILANE DENDRIMER COMPOUNDS, PROCESS FOR PRODUCING THE SAME AND VEROTOXIN NEUTRALIZERS AND ANTIVIRAL AGENTS**

(57) A carbosilane dendrimer compound containing sugar chain in its chemical structure that show neutralizing activity against verotoxin and antiviral activity, and a process for producing the same are provided. Further, a carbosilane dendrimer compound that contains sialyl lactose at its terminus, which can specifically bind and adhered to viruses, is provided.

*Fig. 1*

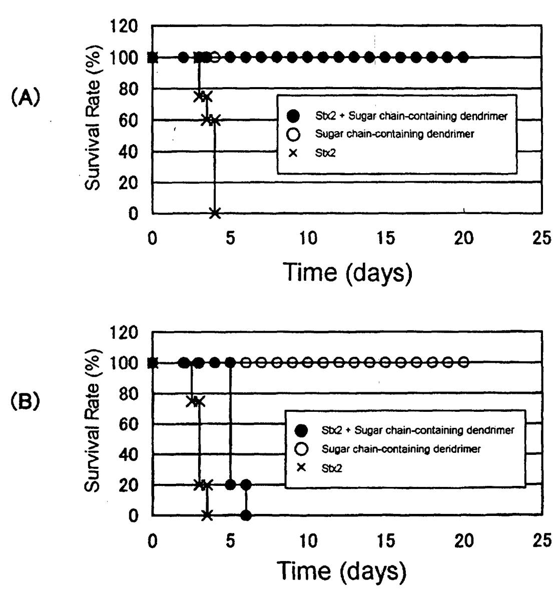


EP 1 302 475 A1

## Description

Technical Field

The invention of the present application relates to a sugar chain-containing carbosilane dendrimer compound that shows antiviral activity and neutralizing activity against verotoxin, and to a method for producing the same. The invention of the present application also relates to a sugar chain-containing carbosilane dendrimer compound that contains terminal sialyl lactose to which viruses are specifically bonded, and to a process for producing the same.

Background Art

Verotoxin, which is produced by enterohemorrhagic *Escherichia coli* 0-157 is a protein belonging to the $AB_5$ family of bacterial toxin similar to shigatoxin derived from *Shigella dysenteriae*. It has been known that such toxins recognize and adhere to the globotriaose moiety in globotriaosyl ceramide ($Gb_3$; Galα1-4Galβ1-4Glcβ1-Cer) on renal cells and are incorporated into the cells to exhibit toxicity.
Therefore, research and development concerning substances that effectively inhibit the adhesion process of the toxin and neutralize the toxin have been vigorously carried out.
Among such substances that show neutralizing activity against verotoxin, carbosilane dendrimers that uniformly contain globotriaose with Si as branching points have been known.
However, such carbosilane dendrimers that have previously been reported show insufficient neutralizing activity against verotoxin and were of no practical value. Therefore, a novel carbosilane dendrimer with higher neutralizing activity against verotoxin, for which molecular designing is easier, and show potential for further development, has been desired.
On the other hand, viruses such as the influenza virus contain various proteins on their surface; infection takes place when such proteins recognize and adhere to sugar chains or the like in living organism. As antiviral agents, substances that inhibit these proteins are common and various types are known.
For example, on the surface of the influenza virus, there are two proteins, sialidase that release sialic acid and hemagglutinin that recognize sialyllactose. Conventional antiviral agents for influenza are sialidase inhibitors, which inhibit the action of sialidase.
Further, other than substances that suppress the infection of viruses to living organisms by inhibiting the action of the protein on the surface of viruses, substances that specifically recognize and adhere to such proteins are thought to be effective as antiviral agents. Such substances may be expected to be useful not only as pharmaceuticals that are administered, but as highly effective virus removing filter packing, as well.
From such viewpoint, the inventors of the present application focused on sialyllactose as a substance that specifically adheres to hemagglutinin on the surface of viruses such as the influenza virus, and as a substance that can prevent viral infection. However, a sialyllactose derivative with a molecular structure that enables efficient adhesion to viruses has not been known.
Accordingly, the invention of the present patent application has been made in view of the above problems, and the object of the present invention is to provide a novel carbosilane dendrimer compound which enables controlling the molecular weight, shape and cumulative efficiency by molecular designing, and shows high neutralizing and antiviral activity against verotoxin; further, the object of the present invention is to provide a carbosilane dendrimer compound that is a sialyllactose-containing substance.

Disclosure of the Invention

As a means to solve the above-described problems, the present invention firstly provides a sugar chain-containing carbosilane dendrimer represented by the following formula (I) :

$$(R^1)_m-Si\left\{-R^2-Si\,[R^6]_l,\ \ [R^3-Si-(R^7)_k,\ \ (R^4-S-R^5-A)_{3-k}\,]_{3-l}\right\}_n \qquad (I)$$

(wherein, $R^1$, $R^6$ and $R^7$ may be the same or different and are hydrocarbon groups that may contain a substituent; $R^2$, $R^3$, $R^4$ and $R^5$ may be the same or different and are hydrocarbon chains that may contain a substituent; A is a sugar chain; m is a number selected from 0 to 3; n is a number selected from 4 to 1; m + n - 4; and k and 1 are the same or different numbers selected from 0 to 2).

Secondly, the present invention provides a sugar chain-containing carbosilane dendrimer represented by the following formula (II):

$$(R^1)_m\text{-Si}\,[—R^2\text{-Si}(R^6)_l\,(R^4—S—R^5—A)_{3-l}]_n \qquad \text{(II)}$$

(wherein, $R^1$ and $R^6$ are hydrocarbon groups that may contain a substituent; $R^2$, $R^4$ and $R^5$ may be the same or different and are hydrocarbon groups that may contain a substituent; A is a sugar chain; m is a number selected from 0 to 3; n is a number selected from 4 to 1; m + n = 4; and 1 is a number selected from 0 to 2).

The present invention thirdly provides the above sugar chain-containing carbosilane dendrimer, wherein $R^1$ is a phenyl group, m is 1 and n is 3.

Further, the present invention fourthly provides the above sugar chain-containing carbosilane dendrimer, wherein $R^1$ is a methyl group and m and n are both 2.

The present invention provides, fifthly, any one of the above sugar chain-containing carbosilane dendrimer, wherein the sugar chain A is represented by the following formula (III) :

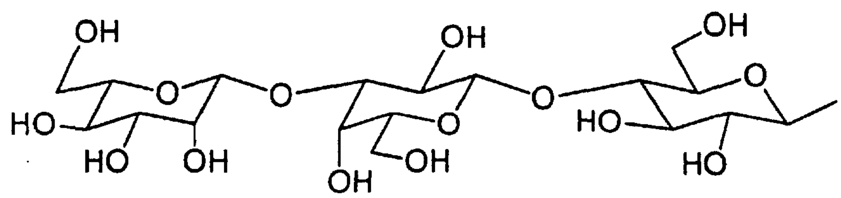

(III)

and sixthly, the any one of the above sugar chain-containing carbosilane dendrimer, wherein the sugar chain A is represented by the following formula (IV):

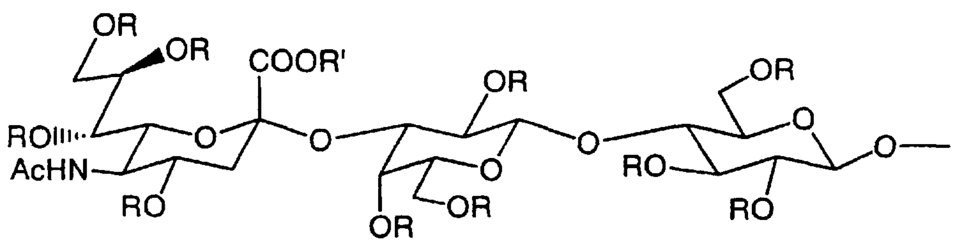

(IV)

(wherein, R is a hydrogen atom or an acetyl group and $R^1$ is a hydrogen atom or a methyl group).

The present invention seventhly provides a method for producing the above sugar chain-containing carbosilane dendrimer, comprising
reacting a halogenated compound represented by the following formula (V):

$$(R^1)_m-\text{Si}\left\{—R^2-\text{Si}\,[R^6]_l,\quad [\,R^3—\text{Si}—(R^7)k,\quad (R^4—X)_{3-k}\,]_{3-l}\right\}_n \qquad \text{(V)}$$

(wherein, $R^1$, $R^2$, $R^3$, $R^4$, $R^6$, $R^7$, m, n, k and l are as defined above and X is halogen atom) with a sulfide compound represented by the following formula (VI):

$$A—R^5—S—Y \qquad \text{(VI)}$$

(wherein, $R^5$ and A are as defined above and Y is a protective group that is released upon reaction).

Furthermore, eighthly, the present invention provides a method for producing the above sugar chain-containing carbosilane dendrimer, comprising

reacting a halogenated compound represented by the following formula (VII):

$$(R^1)_m-Si\left[-R^2-Si(R^6)_l\quad(R^4-X)_{3-l}\right]_n \qquad (VII)$$

(wherein, $R^1$, $R^2$, $R^4$, $R^6$, m, n and l are as defined above and X is a halogen atom) with a sulfide compound represented by the following formula (VI):

$$A-R^5-S-Y \qquad (VI)$$

(wherein, $R^5$ and A are as defined above and Y is a protective group that is released upon reaction).

Ninthly, the present invention provides the method for producing sugar chain-containing carbosilane dendrimer, wherein A in formula (VI) is represented by the following formula (III):

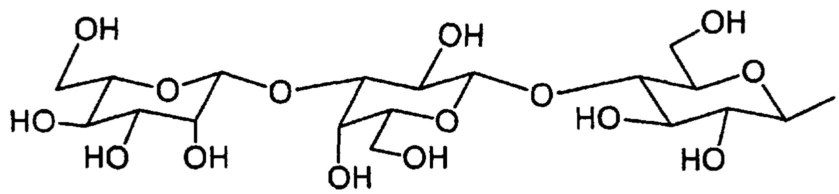

(III)

Further, tenthly, the present invention provides the method for producing sugar chain-containing carbosilane dendrimer, wherein A in formula (VI) is represented by the following formula (IV):

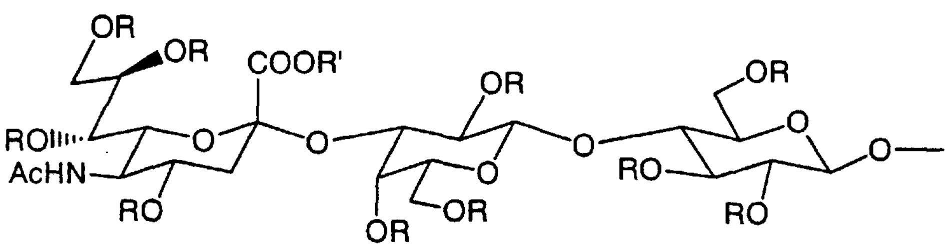

(IV)

Still further, eleventhly, the present invention provides the above ninth and tenth feature of the present invention, wherein Y in formula (VI) is an acetyl group.

The present invention provides, twelfthly, the method for producing the above sugar chain-containing carbosilane dendrimer of the tenth feature of the invention, wherein a thiomethyl glycoside compound of N-acetylneuraminic acid and a trimethylsilyl ethyl glycoside 3',4'-diol compound of lactose are subjected to glycosidation to produce the sialyl lactose derivative of the following formula (VIII):

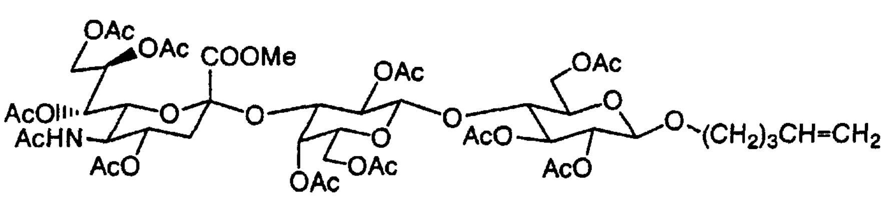

(VIII)

to which thioacetic acid is added by radical addition reaction, thereby producing the acetylthio compound represented by the following formula (IX)

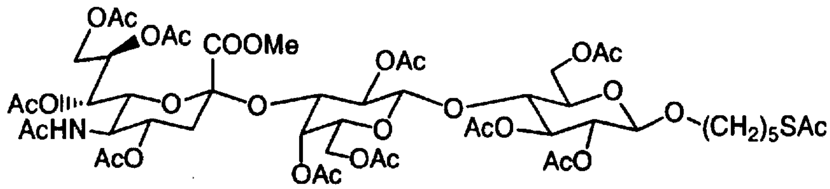

(IX)

to which the carbosilane dendrimer of formula (V) or (VII) are condensated.

And present invention provides, thirteenthly, a neutralizing agent for verotoxin, comprising any of the above sugar chain-containing carbosilane dendrimer as an effective ingredient, fourteenthly, an antiviral agent comprising the same ingredient as an effective ingredient.

Brief Description of the Drawings

Fig. 1 show the survival rates of mice in the Example of the present invention; when the compound of the present invention was solely administered to mice at 50 µg/g body weight (○), when Stx2 alone was administered at a lethal dose (0.25 ng/g body weight) (×) and when Stx2 (0.25 ng/g body weight) was administered together with each of the compounds of the present invention (50 pg/g body weight) (●).

Fig. 2 shows the survival rates of mice in the Examples of the present invention; when Stx2 at a lethal dose (0.25 ng/g body weight) and each concentrations (1.5, 5, 15 and 50 µg/g body weight) of the compound of the present invention were administered (●: 1.5 µg/g body weight; Δ: 5.0 µg/g body weight; ○: 15 µg/g body weight; □: 50 µg/g body weight; ×: no compound added).

Best Mode for Carrying Out the Invention

Features of the present invention are as described above; hereinafter, further embodiments are described.

In any of the compounds (I) and (II) provided by the present invention, the signs $R^2$, $R^3$, $R^4$ and $R^5$ represent identical or different hydrocarbon chains, which may contain substituents; the hydrocarbon chains may be selected from linear or branched aliphatic hydrocarbon chains, cyclic aliphatic hydrocarbons, and aromatic hydrocarbons. Among these hydrocarbons, alkylene chains are typical. For example, $-(CH_2)_r-$ (wherein r = 2 to 6) may be listed. The hydrocarbon groups $R^1$, $R^6$, and $R^7$, which may each contain a substituent, may also be selected from the same hydrocarbon groups as described above; for example, alkyl groups, cycloalkyl groups, aryl groups, and arylalkyl groups may be mentioned.

The hydrocarbon chains or hydrocarbon groups may contain an appropriate substituent. Such substituents include, for example, alkoxy groups, and ester groups.

The sugar chain represented by the sign A is a linking structure moiety comprising a sugar molecule and may be of various structure. Specifically, A may be a globotrisaccharide expressed by the following formula (III) or a sialyllactose expressed by formula (IV):

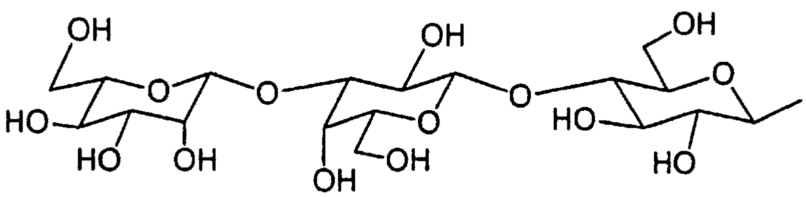

(III)

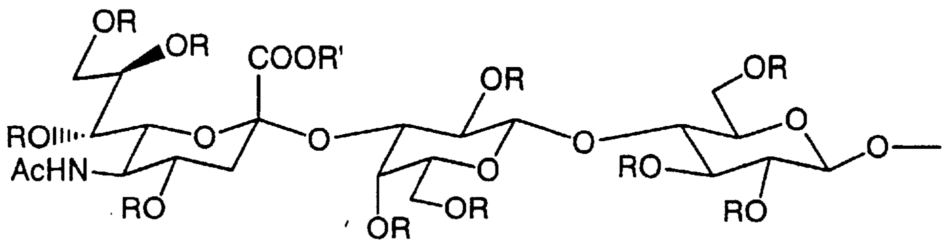

(IV)

The sugar chain A may be linked to the hydrocarbon group $R^5$ via a carbon-carbon bond as shown in formula (III), or via various types of hetero atoms such as in the case for ether bonds as shown in formula (IV).

Examples of the structures of compounds (I) and (II) of the present invention may include the following:

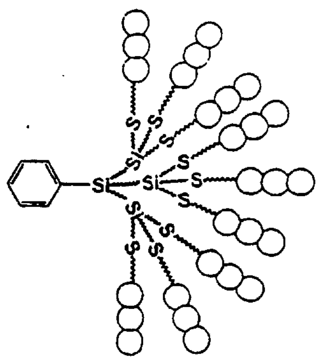

①

Compound (II)

m = 1, n = 3, 1 = 0

Abbr.: Fan(1)9

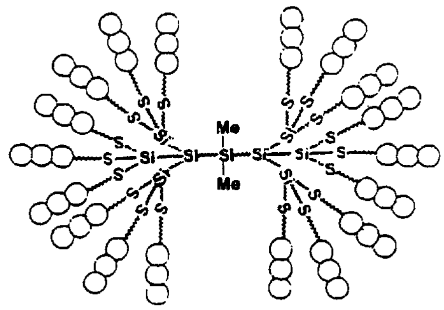

②

Compound (I)

m = 2, n = 2, k = 0, 1 = 0

Abbr.: Dumbbell(2)18

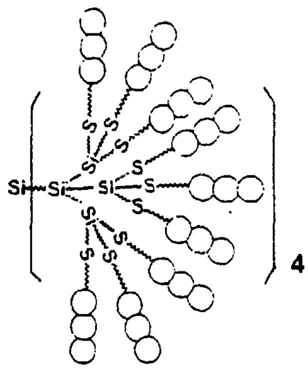

③

Compound (I)

m = 0, n = 4, k = 0, 1 = 0

Abbreviation: Ball(2)36

The wavy lines in the above formulas indicate hydrocarbon chains such as $R^2$, $R^4$, $R^5$ or the like, and may be, for example, alkylene chains such as $-(CH_2)_4-$ or $-(CH_2)_3-$ . Further, the circles (O) indicate various sugar chains such as those represented by the above-mentioned formulas (III) and (IV).

Among the above examples of the compounds, the Fan type carbosilane dendrimer compound wherein the sugar moiety contain 9 sugar chains (1) (abbreviated as: Fan(1)9) is an example of compound (II), in which $R^1$ is a phenyl group, m is 1, n is 3 and 1 is 0; the dumbbell type carbosilane dendrimer wherein the sugar moiety contain 18 sugar chains (2) (abbreviated as: dumbbell(2)18) is an example of compound (I), in which $R^1$ is a methyl group, m is 2, n is 2, and k and 1 are both 0; and the ball type carbosilane dendrimer compound wherein the sugar moiety contain 36 sugar chains (3) (abbreviated as: ball(2)36) is an example of compound (I), in which m is 0, n is 4, and k and l are both 0.

Compounds (I) and (II) of the present invention may be produced by reacting the halide (V) or (VII) with the sulfide compound (VI), as described above. Here, as the sugar chain A, compounds expressed by the above formulas (III) or (IV) may be exemplified. Further, in the sulfide compound (IV), the protecting group Y that is released upon reaction may preferably be an acetyl group.

To describe the process in more detail, for example, the following steps may be employed.

<1> Synthesis of carbosilane dendrimer skeleton

Using chlorosilane as the starting material, the skeleton of compound (I) or (II) is formed by introducing an allyl group and repeating the hydrosilation reaction and the Grignard reaction. The terminal allyl group of the resultant dendrimer is converted to the corresponding alcohol by hydroboration reaction, followed by mesylation by conventional methods, and converted to halides wherein the end-groups are substituted by bromine atom, by processing with sodium bromide.

For example, by these methods, the above-described compounds (V) or (VII) may be prepared. The halogen atom (X) may be bromine originated from sodium bromide as described above or others such as chlorine or iodine.

<2> Formation of sugar chain derivatives

Globotriaose (III) derivative may be synthesized, for example, as follows. Namely, the globotrisaccharide skeleton is synthesized through the one-step glycosidation from sugar donors and sugar acceptors derived from n-butenyl β -glycoside originating from D-galactose and D-lactose, respectively. Further after being subjected to various types of protection and deprotection reactions, the radical addition of benzyl mercaptan to the butenyl group, followed by the release of the protecting group gives a precursor for bonding with the dendrimer.

For example, compound (VI) may be prepared, by such procedures. The protecting group (V) that is released upon reaction may be a benzyl group as described above, or any other group.

On the other hand, sialyllactose (IV) derivatives may be synthesized by the following procedures.

Namely, using a thiomethyl glycoside of N-acetyl neuraminic acid synthesized by known methods (A. Hassegawa et al., Carbohydr. Res., 212, 277-281 (1991)) as a sialic acid donor and trimethylsilylethyl glycoside-3',4'-diol synthesized by known methods (K.P.R. Kartha, etal., J. Carbohydr. Chem., 8, 145-158 (1989)) as a lactose acceptor, the two compound are glycosidated, followed by substituting a pentenyl group as the aglycone, gives a precursor (sialyl lactose derivative) for bonding to with dendrimer.

<3> Binding of carbosilane dendrimer skeleton and sugar chain

The one pot method in liquid ammonia developed by the present inventors can be used for this purpose. Namely, thioanion is first prepared by the Birch reduction of the benzyl sulfide derivative originated from globotriaose, and then subjected to SN2 reaction with the bromine atom on the dendrimer to obtain the target compound (1) or (II).

For introduction of sialyllactose (IV) into carbosilane dendrimer, for example, the radical addition of thioacetic acid with the above-described sialyllactose derivative gives the acetylthio derivative of formula (VIII), which is then subjected to condensation with the carbosilane dendrimer containing halogen atoms such as bromine and chlorine at its terminus. By subjecting the resultant compound to deprotection, the carbosilane dendrimer of formula (IV), wherein R and R' are both hydrogen atoms can be obtained.

In the above-described production method, each step of the reaction is performed by applying known techniques for chemical experiments such as oxidization, reduction, addition, condensation, substitution, protection, deprotection, and ion exchange. The successful synthesis of the dendrimer containing the sialyl lactose (IV) at its terminus has never been reported. Reaction conditions such as type of solvent, temperature and pressure for each reaction steps are not limited.

Thus, the sugar-containing carbosilane dendrimer of the present invention enables relatively easy molecular designing and synthesis of its carbosilane dendrimer moiety, and allows the adjustment of the molecular size, density (packing) of the dendric subunit, and the number of sugar chains in accordance with its use. In addition, the compound of the present invention is characteristic in that by changing the sugar chain moiety, the target can be changed in a wide range. Further the sugar-containing carbosilane dendrimer of the present invention has low toxicity because of its structural features.

Furthermore, compounds (I) and (II) of the present invention shows neutralizing activity against verotoxin produced by *Escherlchia coli* O-157. The dumbbell type compound shows especially high neutralizing activity against verotoxin. These compounds can also be used as inhibitors against viruses such as the influenza virus and the AIDS virus.

The carbosilane dendrimer containing sialyllactose (IV) as the sugar chain A, which has been synthesized for the first time, can be used as a substance that allows the efficient adhesion of viruses, since sialyllactose can specifically bind to viruses. Therefore, when ingested, the compound acts as an effective ingredient of an antiviral agent for preventing infection of the viruses; further, the compound can be used as packing for filters for the specific adhesion and removal of viruses from the environment including air.

The present invention is described in further detail with reference to the following Examples. It should be needless to mention that the present invention is not limited to the following Examples, and embodiments with various modifications are possible.

Examples

<Reference Example 1> Synthesis of carbosilane dendrimer containing bromine atom at its terminus (A)

A carbosilane dendrimer containing bromine atoms at its terminus was synthesized in accordance with the following reaction formula.

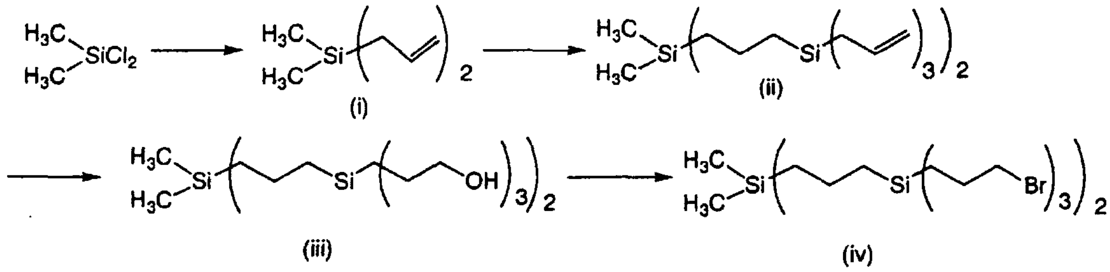

(1) Diallyldimethylsilane (compound i)

Dichlorodimethylsilane (0.40 mL, 77.5 mmol) was dissolved in distilled diethyl ether (20 mL) under argon, after which a 1M solution of allyl magnesium bromide in diethyl ether (232 mL, 232 mmol) was added dropwise while cooling over ice, followed by stirring at 50°C for 8 hours. After completion of the reaction, 1N hydrochloric acid (about 150 mL) was added while cooling over ice, and extracted with diethyl ether; the extract was then washed with distilled water. The organic layer was dried with anhydrous sodium sulfate, filtrated and concentrated. The residue was purified by vacuum distillation (54 mmHg/58°C) and compound i (7.48 g, 68.8%) was obtained as a liquid.

Identification results are shown in Table 1.

Table 1

| |
|---|
| $^1H$ NMR δ (200 MHz, $CDCl_3$), 0.01 (s, 6H, 2Me), 1.54 (m, 4H, $2SiCH_2CH=CH_2$), 5.23 (m, 4H, $2SiCH_2CH=CH_2$), 5.78 (m, 2H, $2SiCH_2CH=CH_2$) |

(2) Bis(triallylsilylpropyl)dimethylsilane (compound ii)

Compound i (7.00 g; 49.9 mmol) was dissolved in distilled THF (50 mL) under argon and a catalytic amount of Speier catalyst (a 0.1 M solution of hexachloroplatinic (IV) acid hexahydrate in isopropanol) was added dropwise. Next, trichlorosilane (20.1 mL, 200 mmol) was added dropwise to the reaction solution while cooling over ice and the dropping funnel was washed with distilled THF (30 mL) . After stirring at room temperature for 18.5 hours, the reaction solution was distilled (75-80°C) under standard pressure to evaporate the solvent and the excessive trichlorosilane. Distilled THF (60 mL) was added, and a 1 M solution of allyl magnesium bromide in diethyl ether (645 mL, 645 mmol) was added dropwise while cooling over ice; the mixture was stirred at 0°C for 1 hour, at room temperature for 1.5 hours and at 50°C for 18 hours. After completion of the reaction, 1N hydrochloric acid (about 500 mL) was added while cooling over ice, and extracted with diethyl ether; the extract was then washed with distilled water. The organic layer was dried over anhydrous sodium sulfate and the solution was filtrated and concentrated. The residue was purified by silica gel chromatography (hexane only) to give compound ii (8.33 g, 37.5%) as a liquid.

Identification results are shown in Table 2.

Table 2

| |
|---|
| $^1H$ NMR δ (200 MHz, $CDCl_3$), -0.05 (s, 6H, 2Me), 0.64 (m, 8H, $2SiMe_2CH_2CH_2CH_2Si$), 1.33 (m, 4H, $2SiMe_2CH_2CH=CH_2$), 1.59(m, 12H, $6SiCH_2CH=CH_2$), 4.88 (m, 12H, 6 $SiCH_2CH=CH_2$), 5.80 (m, 6H, 6 $SiCH_2CH=CH_2$) |

(3) Bis[tris(3-hydroxypropyl)silylpropyl]dimethyl- silane (compound iii)

Compound ii (3.00 g, 6.74 mmol) was dissolved in distilled THF (90 mL) under argon and a 1.0 M solution of a $BH_3$-THF complex in THF (27.0 mL, 27.0 mmol) was added dropwise while cooling over ice; the mixture was stirred at room temperature for 20 hours. Then, distilled water (7.28 mL; 405 mmol) was added dropwise while cooling over ice, and a 3.0 M aqueous solution of sodium hydroxide (8.99 mL, 27.0 mmol) and a 30% aqueous solution of hydrogen peroxide (9.17 mL, 80.9 mmol) were added consecutively; the mixture was stirred at room temperature for 2 hours. After completion of the reaction, a solution of ferrous (II) sulfate heptahydrate (22.5 g, 80.9 mmol) dissolved in a small amount of water was added while cooling over ice, followed by stirring for 1 hour. The reaction solution separated into two layers and the upper layer was decanted to an Erlenmeyer flask, to which sodium chloride was added to separate the THF layer and the aqueous layer, and the THF layer was washed with a saturated saline solution. The organic layer was dried over anhydrous sodium sulfate and the solution was filtrated and concentrated. The residue was purified by silica gel column chromatography (a 6:1 mixture of chloroform and methanol) to give compound iii (1.82 g, 48.9%) as a liquid.

Identification results are shown in Table 3.

Table 3

| |
|---|
| $^1H$ NMR δ (200 MHz, DMSO-$d_6$), -0.12 (s, 6H, 2Me), 0.44 (m, 20H, $2SiMe_2CH_2CH_2CH_2Si$, $6SiCH_2CH_2CH_2OH$), 1.31(m, 16H, $2SiMe_2CH_2CH_2CH_2Si$, $6SiCH_2CH_2CH_2OH$), 3.28 (t, 12H, $6SiCH_2CH_2CH_2OH$) |
| Anal. Calcd for $C_{26}H_{60}O_6Si_3$(553.01): C, 56.47; H, 10.94. Found C, 56.21; H, 11.10 |

Compound iii was then mesylated to give bis[tris(3-bromopropyl)silylpropyl]dimethylsilane (compound iv: 420 mg, 55.0%) as a liquid. Identification results are shown in Table 4.

Table 4

IR(neat) 1239 cm·1 ($CH_2Br$)
$^1$H NMR δ (200 MHz, $CDCl_3$), ·0.02 (s, 6H, 2Me), 0.62 (m, 20H, $2SiMe_2CH_2CH_2C\mathit{H_2}Si$, 6 $SiC\mathit{H_2}CH_2CH_2Br$), 1.30 (m, 4H, $2SiMe_2CH_2C\mathit{H_2}H_2Si$) 1.81(m, 12H, $6SiCH_2C\mathit{H_2}CH_2Br$), 3.39 (t, 12H, $6SiCH_2CH_2C\mathit{H_2}Br$)

<Reference Example 2> Synthesis of carbosilane dendrimer containing bromine atom at its terminus (B)

In the same manner as described in Reference Example 1, a halide compound with a carbosilane dendrimer skeleton was synthesized according to the following reaction formula.

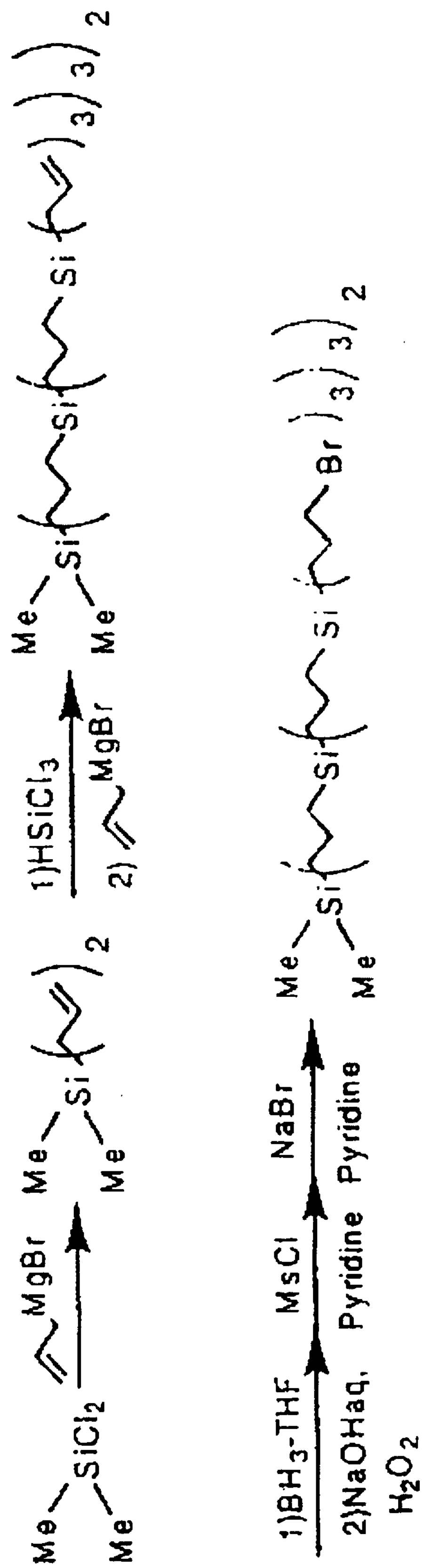
MgBr
1)$HSiCl_3$
2) MgBr
1)$BH_3$-THF
2)NaOHaq, $H_2O_2$
MsCl
Pyridine
NaBr
Pyridine

<Reference Example 3> Synthesis of sugar chain derivative (A)

A benzylsulfide derivative 4 of globotriaose was synthesized according to the following reaction formula.

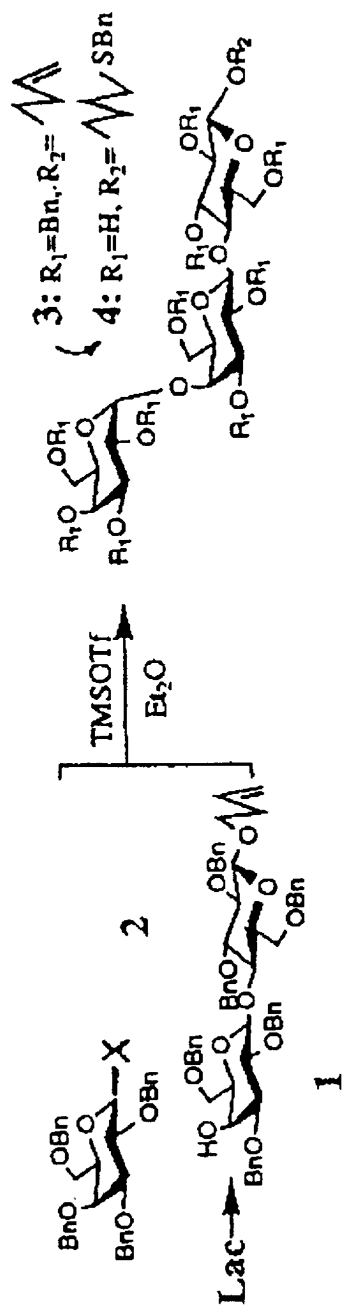


Thus, first, the aglycone of lactose was converted to a butenyl group, which can endure debenzylation that follows, and only the 4'-OH group was derived to become the free compound 1. Then glycosidation was performed with a known galactosyl donor 2 using a catalyst to give trisaccharide derivative 3.

After debenzylation by Birch reduction, the protective group was converted to an acetyl group, and the buteny group was subjected to radical addition with benzyl mercaptan, and O-deacetylated to obtain 4.

<Reference Example 4> Synthesis of sugar chain derivative (B)

A sialyl lactose derivative was synthesized according to the following reaction formula.

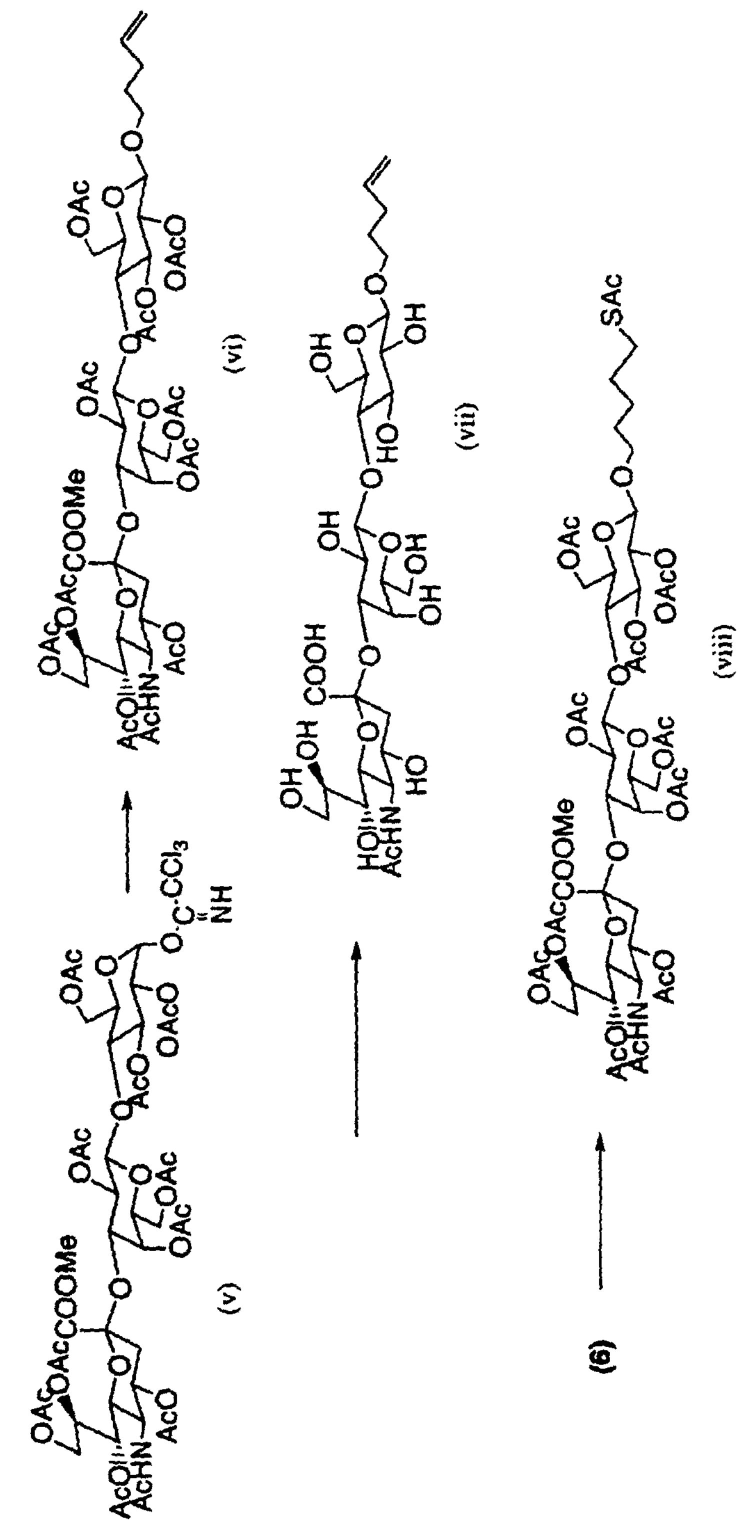

(v) (vi) (6) (vii) (viii)

(1) Pentenyl[methyl(5-acetamido-4,7,8,9-tetra-O-acetyl-3,5-dideoxy-D-glycero-α-D-galacto-2-nonulo-pyranosyl) onate]-(2→3)-O-(2,4,6-tri-O-acetyl-β-D-galacto-pyranosyl)-(1→4)-2,3,6-tri-O-acetyl-β-D-glucopyranoside (compound vi)

An α-trichloroacetoimidate compound (compound v: 1.032 mg, 0.853 mmol) was dissolved in dichloromethane (24.0 mL) and 4-penten-1-ol (0.432 mL, 4.265 mmol) and MS 4A (2.0 g) were added followed by cooling at -25°C. A boron trifluoride ethyl ether complex (215 μL; 1.71 mmol) was added, followed by stirring for 20 minutes; the reaction

solution was returned to -5°C and stirred for another 4 hours. The reaction solution was filtrated, and the Celite filtrate was extracted with a cold saturated aqueous solution of sodium hydrogen carbonate and a saturated saline solution consecutively; the organic layer was dried over anhydrous sodium sulfate. This organic layer was filtrated and the filtrate was concentrated and purified by silica gel column chromatography [chloroform-methanol (20:1 v/v)] to give a foamy trisaccharide pentenyl glycoside derivative (compound vi: 816 mg, 84.3%).

Identification results are shown in Table 5.

Table 5

$R_f$ 0.5 [10:1 (v/v) chloroform-methanol]; $[\alpha]_D^{18}$ −6.865 ° (*c* 2.020, $CHCl_3$);

N. M. R. data : $^1H$ ($CDCl_3$), δ 5.78 (m, 1 H, -*CH*=$CH_2$), 5.54 (m, 1 H, H-8"), 5.39 (dd, 1 H, $J_{6",7"}$ = 2.7 Hz, $J_{7",8"}$ = 9.3 Hz, H-7"), 5.18 (t, 1 H, $J_{2,3}$ = $J_{3,4}$ = 9.3 Hz, H-3), 5.08 (d, 1 H, $J_{NH,5"}$ = 10.2 Hz, *NH*), 5.02–4.85 (m, H-2, H-2', H-4', -CH=$CH_2$), 4.67 (d, 1 H, $J_{1',2'}$ = 10.0 Hz, H-1'), 4.52 (dd, 1 H, $J_{2',3'}$ = 10.2 Hz, $J_{3',4'}$ = 3.3 Hz, H-3'), 4.45 (d, 1 H, $J_{1,2}$ = 8.0 Hz, H-1), 4.43 (m, 2 H, H-6b, H-9"), 4.18 (dd, 1 H, $J_{5,6a}$ = 5.4 Hz, $J_{6a,6b}$ = 11.9 Hz, H-6a), 4.02 (m), 3.87 (m, 3 H, H-4, one of $OCH_2$-, -*CH*), 3.84 (s, 3 H, $COOCH_3$), 3.63 (dd, 1 H, $J_{5",6"}$ = 10.8 Hz, $J_{6",7"}$ = 2.7 Hz, H-6"), 3.59 (m, 1 H, H-5), 3.49 (dt, 1 H, $J_{gem}$ = 9.6 Hz, $J_{vic}$ = 6.7 Hz, one of $OCH_2$-), 2.58 (dd, 1 H, $J_{3"a,3"e}$ = 12.6 Hz, $J_{3"e,4"}$ = 4.5 Hz, H-3"eq), 2.25, 2.16, 2.09, 2.08, 2.06, 2.04, 2.03, 2.01, and 1.86 (each s, 33 H, NH*Ac*, O*Ac*), 2.08 (m, $OCH_2CH_2CH_2CH=CH_2$), 1.66 (m, $OCH_2CH_2$-, H-3"ax).

N. M. R. data : $^{13}C$ ($CDCl_3$), δ 170.66, 170.51, 170.48, 170.45, 170.35, 170.25, 170.17, 170.05, 169.65, 169.54, 169.48, 169.44, 167.81, 137.68, 114.91, 100.82, 100.40, 96.63, 76.19, 73.27, 72.45, 71.86, 71.70, 71.20, 70.30, 69.77, 69.20, 69.13, 67.70, 67.14, 66.78, 62.20, 62.12, 61.37, 52.98, 48.88, 37.25, 29.67, 28.44, 22.99, 21.36, 20.78, 20.67, 20.62, 20.56, 20.52, 20.45.

I. R. data (KBr) : ν ($cm^{-1}$) 2949 (C-H), 1745 (C=O, ester), 1674 (C=O, SAc, amide), 1554 (N-H, amide), 1230 (C-O, ester), 1045 (C-O, ether).

*Anal.* Calc. for $C_{49}H_{69}N_1O_{29}$: C, 51.80; H, 6.12; N, 1.23 %. Found: C, 51.50; H, 6.14; N, 1.19 %.

(2) Pentenyl (5-acetamido-3,5-dideoxy-D-glycero-α-D-galacto-2-nonulopyranosylic acid)-O-β-D-galactopyranosyl-(1→4)-O-β-D-glucopyranoside (compound vii)

Compound vi (50.0 mg, 0.0440 mmol) was dissolved in methanol (1.0 mL). A 1 M sodium methoxide-meth-

anole solution (44 μL) was added followed by stirring at room temperature over night. The reaction solution was neutralized with a strongly acidic cation-exchange resin IR-120B ($H^+$ type) (2 mL), after which the ion-exchange resin was collected by filtration and the filtrate was concentrated. To the residue was added a 0.05M aqueous solution of sodium hydroxide (2 mL) followed by stirring at room temperature for 2 hours. The reaction solution was neutralized using a strongly acidic cation-exchange resin IR-120B ($H^+$ type) (1mL), and the ion-exchange resin was collected by filtration; the filtrate was concentrated to give a sialyllactose derivative (compound vii: 26.0 mg, 84.4%).

Identification results are shown in Table 6.

Table 6

$[\alpha]_D^{18}$ −3.446 ° ($c$ 0.963, $CHCl_3$);

N. M. R. data : $^1$H ($D_2O$), HDO δ 4.70, δ 5.78 (m, 1 H, -$CH$=$CH_2$), 4.96 (dd, 1 H, $J_{gem}$ = 1.5 Hz, $J_{trans}$ = 15.4 Hz, one of -CH=$CH_2$), 4.96 (d, 1 H, $J_{cis}$ = 10.3 Hz, one of -CH=$CH_2$), 4.40 (d, 1 H, $J_{1',2'}$ = 7.7 Hz, H-1'), 4.35 (d, 1 H, $J_{1,2}$ = 8.1 Hz, H-1), 4.03 (dd, 1 H, $J_{2',3'}$ = 9.7 Hz, $J_{3',4'}$ = 2.8 Hz, H-3'), 3.88-3.42 (m), 3.18 (t, 1 H, $J_{2,3}$ = 7.9 Hz, H-2), 2.63 (dd, 1 H, $J_{3''a,3''e}$ = 12.5 Hz, $J_{3''e,4''}$ = 4.0 Hz, H-3"eq), 2.02 (q, 2 H, $J$ = 7.1 Hz, -$CH_2$CH=$CH_2$), 1.91 (s, 3 H, NDAc), 1.77 (t, 1 H, $J_{3''a,4''}$ = 12.1 Hz, H-3"ax), 1.60 (m, 2 H, $OCH_2CH_2CH_2$).

I. R. data (KBr) : ν ($cm^{-1}$) 3394 (O-H), 2937 (C-H), 1732 (C=O,carboxyl acid), 1639 (C=O, amide), 1560 (N-H, amide), 1036 (C-O, alcohol, ether), 619 (N-H, out-of-plane bending)

*Anal.* Calc. for $C_{28}H_{47}N_1O_{19}$-1.5$H_2O$ : C, 46.15; H, 6.92; N, 1.92 %. Found: C, 46.29; H, 6.77; N, 1.82 %.

(3) ω-Acetylthio-pentanyl [methyl (5-acetamido-4,7,8, 9-tetra-O-acetyl-3,5-dideoxy-D-glycero-α-D-galacto-2-nonulopyranosyl)onate]-(2→3)-O-(2,4,6-tri-O-acetyl-β-D-galactopyranosyl)-(1→4)-2,3,6-tri-O-acetyl-β-D-glucopyranoside (compound viii)

In a flask equipped with an air-cooling pipe, compound vi (100 mg, 0.0881 mmol) was dissolved in 1,4-dioxane (209 μL) and thioacetic acid (209 μL, 3.0 mmol) was added followed by heating at 50°C. AIBN (24.6 mg, 0.150 mmol) was added followed by heating at 80°C under stirring. After stirring for 3 hours, the mixture was cooled down to room temperature; cyclohexane (608 μL) was added followed by stirring for several minutes. After adding toluene to the reaction solution, the mixture was subjected to azeotropic concentration, diluted with chloroform and successively extracted with a cold saturated aqueous solution of sodium hydrogen carbonate and saturated saline solution consecutively; the organic layer was dried over anhydrous sodium sulfate. This organic layer was filtrated and the filtrate was concentrated and purified by silica gel column chromatography [chloroform 100% → chloroform-methanol (30:1 v/v)] to give a trisaccharide-ω-acetylthiopentyl glycoside derivative (compound viii; 105 mg; 99.1%).

Identification results are shown in Table 7.

Table 7

$R_f$ 0.51 [10:1 (v/v) chloroform-methanol]; $[\alpha]_D^{29}$ −6.276 ° (*c* 1.29, $CHCl_3$);

N. M. R. data : $^1H$ ($CDCl_3$), δ 5.49 (m, 1 H, H-8"), 5.39 (dd, 1 H, $J_{6'',7''}$ = 2.8 Hz, $J_{7'',8''}$ = 9.4 Hz, H-7"), 5.18 (t, 1 H, $J_{2,3}$ = $J_{3,4}$ = 9.4 Hz, H-3), 5.12 (d, 1 H, $J_{NH,5''}$ = 9.9 Hz, *NH*), 4.93 (dd, 1 H, $J_{1',2'}$ = 8.1 Hz, $J_{2',3'}$ = 10.3 Hz, H-2'), 4.88 (m, H-2, H-4", -*CH*), 4.67 (d, 1 H, H-1'), 4.52 (dd, 1 H, $J_{3',4'}$ = 3.3 Hz, H-3'), 4.45 (d, 1 H, $J_{1,2}$ = 7.7 Hz, H-1), 4.43 (m, H-6b, H-9", -*CH*), 4.18 (dd, 1 H, $J_{5a,6b}$ = 11.9 Hz, $J_{5,6a}$ = 5.4 Hz, H-6a), 4.09-3.82 (2m), 3.84 (s, 3 H, COO*CH*$_3$), 3.63 (dd, 1 H, $J_{5'',6''}$ = 10.7 Hz, $J_{6'',7''}$ = 2.6 Hz, H-6"), 3.59 (m, 1 H, H-5), 3.46 (dt, 1 H, $J_{gem}$ = 9.7 Hz, $J_{vic}$ = 6.6 Hz, one of O*CH*$_2$-), 2.85 (t, 2 H, *J* = 7.2 Hz, S*CH*$_2$-), 2.58 (dd, 1 H, $J_{3''a,3''e}$ = 12.7 Hz, $J_{3''e,4''}$ = 4.6 Hz, H-3"eq), 2.32 (s, 3 H, S*Ac*), 2.24 (s, 3 H, NHAc), 2.16, 2.094, 2.086, 2.086, 2.077, 2.061, 2.040, 2.040, 2.008, and 1.855 (each s, 30 H, 10 O*Ac*), 1.61 (m, 5 H, H-3"ax, $OCH_2$*CH*$_2$$CH_2$*CH*$_2$$CH_2S$), 1.39 (m, 2 H, -*CH*$_2$$CH_2CH_2SAc$).

I. R. data (KBr) : ν ($cm^{-1}$) 2959 (C-H), 1756 (C=O, ester), 1689 (C=O, SAc, amide), 1544 (N-H, amide), 1436 (C-N), 1250 (C-O, ester), 1040 (C-O, ether), 631-603 (N-H, out-of-plane bending)

FAB-MS *Anal.* Calc. for $C_{51}H_{73}N_1O_{30}S_1$ $[M+H]^+$ : 1212, $[M+Na]^+$ : 1234. Found : $[M+H]^+$ : 1212, $[M+Na]^+$ : 1234.

*Anal.* Calc. for $C_{51}H_{73}N_1O_{30}S_1$: C, 50.53; H, 6.07; N, 1.16 %. Found: C, 50.03; H, 6.03; N, 1.14 %.

<Example 1> Synthesis of sugar chain-containing carbosilane dendrimer compound

(1) The benzyl sulfide derivative 4 prepared in Reference Example 3 was subjected to Birch reduction to produce a thioanion and immediately subjected to an SN2 reaction in liquid ammonia with the dendrimer halide compound containing Br atom synthesized in the above Reference Example 2, whereupon a sugar chain-containing carbosilane dendrimer compound (compound a of the following formula) with a dumbbell structure containing 18 globotriaosides was prepared.

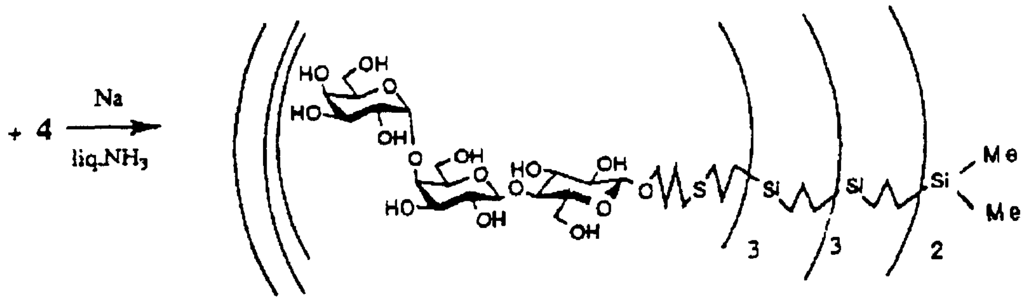


a

Physical properties of this compound a abbreviated as "dumbbell(2)18" are shown in Table 8.

Table 8

MaDLI-TOF-MAS $[M+Na]^+$

Found: 12049.9

Calcd. : 12048.6

IR (KBr) ($cm^{-1}$)

3361 ($\nu_{0\text{-}H}$), 2915 ($\nu_{C\text{-}H}$), 1420 ($\nu_{Si\text{-}C}$), 1274 ($\nu_{C\text{-}0\text{-}C}$), 706 ($\nu_{Si\text{-}C}$)

Compound a was identified from the result of H-NMR ($D_2O$) and C-NMR ($D_2O$) together with the above-mentioned physical property data.

(2) Similarly, a sugar chain-containing carbosilane dendrimer compound (compound b) with a ball-type structure containing 36 globotriaosides was synthesized in accordance with the following reaction formula.

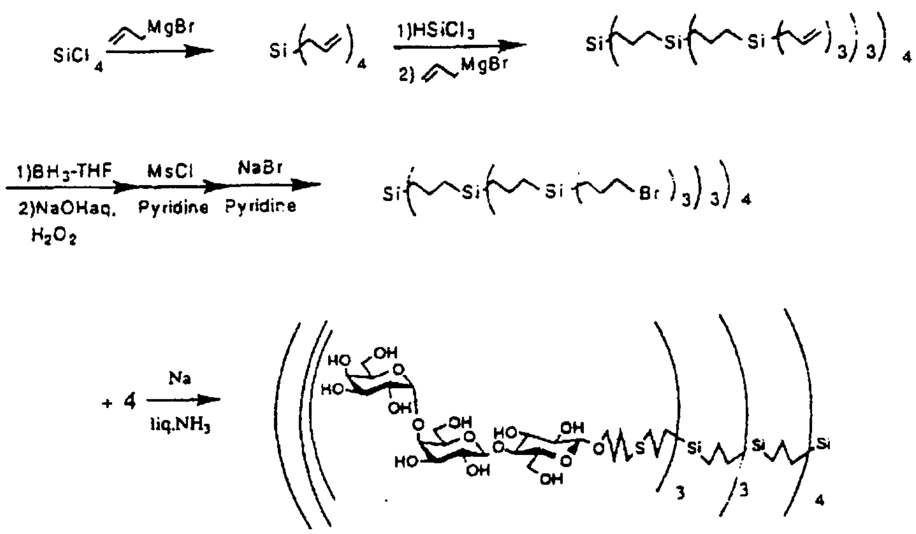


b

Physical properties of this compound b abbreviated as "ball(2)36" are shown in Table 9.

Table 9

MaDLI-TOF-MAS $[M+Na]^+$
Found: 23988.2
Calcd. : 23986.1
IR (KBr) ($cm^{-1}$)
3402.1 ($\nu_{0-H}$), 2914 ($\nu_{C-H}$), 1417 ($\nu_{Si-C}$), 1076 ($\nu_{C-O-C}$), 702 ($\nu_{Si-c}$)
CNMR ($D_2O$)
$\delta$ = 103.41, 102.5, 100.46, 78.71, 77.54, 75.60, 74.95, 74.71, 73.13, 72.40, 71.04, 70.99, 69.97, 69.31, 69.10, 68.75, 60.72, 60.58, 60.43, 35.95, 31.84, 28.81, 26.08, 24.44,
GI (23.39, 19.29, 19.05), GO (18.02, 15.26, 13.53), 12.12

For the identification of compound b, analytical values by H-NMR ($D_2O$) were also used.
(3) Similarly, a sugar chain-containing carbosilane dendrimer compound (compound c of the following formula) with a fan-type structure containing 9 globotriaosides was synthesized.

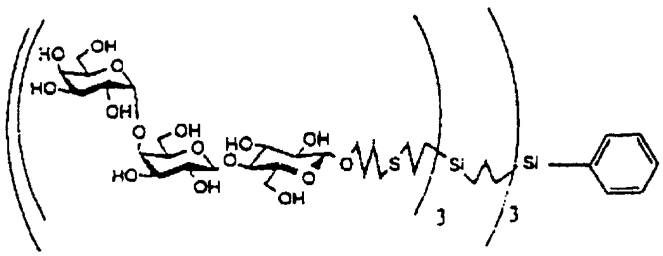

c

Physical properties of this compound c abbreviated as "fan(2)9" are shown in Table 10.

Table 10

**FAB-MAS $[M+H]^+$**
Found: 6019.7
Calcd. : 6019.3
IR (KBr) ($cm^{-1}$)
3393 ($\nu_{0-H}$), 2915 ($\nu_{C-H}$), 1419 ($\nu_{Si-C}$), 1073 ($\nu_{C-O-C}$), 707 ($\nu_{Si-C}$)
CNMR ($D_20$)
$\delta$ = 103.48, 102.54, 100.55, 78.78, 77.60, 75.68, 75.01, 74.81, 73.26, 72.46, 71.14, 70.05, 69.51, 69.27, 69.05, 68.71, 60.77, 60.65, 60.42, GI (35.88, 31.84, 28.89, 26.12, 24.51),
G0 (21.07, 18.94, 17.74), 12.11

(4) Similarly, a sugar chain-containing carbosilane dendrimer compound (compound d of the following formula) with a dumbbell type structure containing 4 globotriaosides was synthesized.

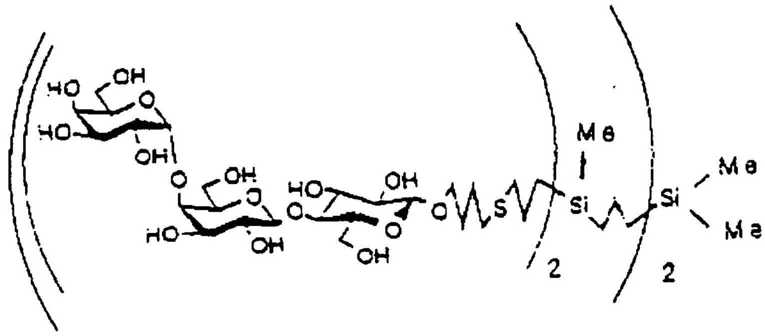

d

Physical properties of this compound d abbreviated as "dumbbell(1)4" are shown in Table 11.

Table 11

FAB-MAS $[M+H]^+$
Found: 2764.1
Calcd. : 2764.1
IR (KBr) ($cm^{-1}$)
3400 ($\nu_{0\text{-}H}$), 2911 ($\nu_{C\text{-}H}$), 1419 ($\nu_{Si\text{-}C}$), 1073 ($\nu_{C\text{-}0\text{-}C}$), 705 ( $\nu_{Si\text{-}C}$)
CNMR ($D_20$)
$\delta$ = 103.43, 102.57, 100.49, 78.71, 77.55, 75.63, 74.96, 74.74, 73.14, 72.42, 71.05, 71.05, 69.92, 69.34, 69.12, 68.76, 60.74, 60.60, 60.44, GI (35.87, 31.81, 28.84, 26.92, 24.45),
G0 (20.32, 18.80, 18.80), GI (13.30), Me (-2.31, -4.43)

Identification of compound d was carried out by H-NMR, as well.

<Example 2>

Various sugar chain-containing carbosilane dendrimer compounds were synthesized according to the following reaction formula.

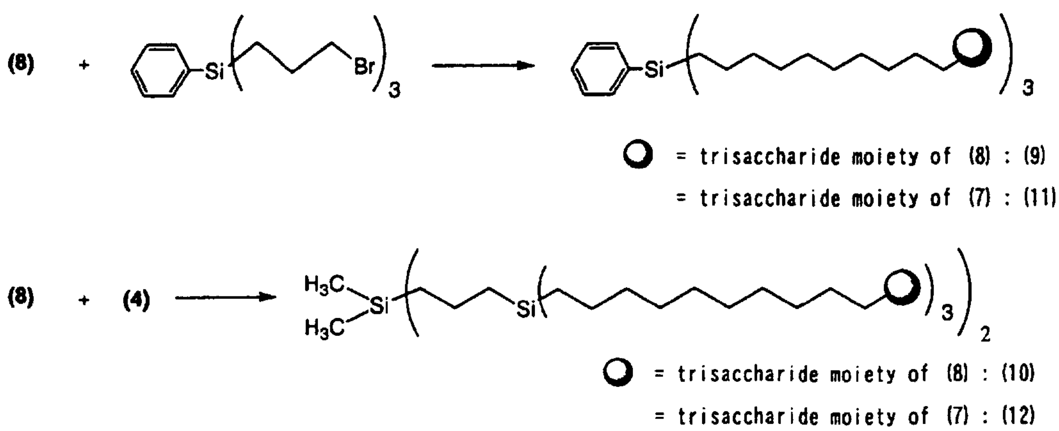


(C)

(1) Fan-(0)3-2,3-SLac-Ac (compound ix)

Fan(0)3-Br dendrimer (abbreviated) (6.48 mg, 0.013748 mmol) and a trisaccharide omega-acetylthiopentanal glycoside derivative (compound viii: 100 mg, 0.08249 mmol) were dissolved in DMF (0.2 mL) until a homogeneous system was obtained. After dissolving, methanol (0.2 mL) was added followed by stirring at room temperature for 1

hour. Sodium methoxide (4.90 mg) was added followed by stirring over night, after which acetic acid (0.1 mL) was added followed by stirring for several minutes and subjecting to concentration. After concentration, acetic anhydride (2 mL) and pyridine (2 mL) were added followed by stirring; after completion of acetylation, the reaction was concentrated and extracted with cold 1 M hydrochloric acid, saturated saline solution and chloroform, consecutively; the organic layer was dried over anhydrous sodium sulfate. The organic layer was filtrated, and the filtrate was concentrated and dissolved in methanol and diethyl ether, followed by the dropwise addition of a solution of diazomethane in ether. Acetic acid was added to the reaction solution and the mixture was concentrated, purified by silica gel column chromatography [chloroform-methanol (30:1 v/v), 20 mL of flush silica gel] and further subjected to purification by gel filtration using Sephadex LH-20 (MeOH) to give fan(0)3-2, 3-SLac-Ac (compound ix: 41.2 mg, 80.2%).

Identification results are shown in Table 12.

Table 12

N. M. R. data : $^1$H (CDCl$_3$), δ 7.40 (m, 5 H, *Ph*), 5.53 (m, 3 H, H-8"), 5.39 (dd, 3 H, $J_{6'',7''}$ = 2.7 Hz, $J_{7'',8''}$ = 9.1 Hz, H-7"), 4.52 (dd, 1 H, $J_{2',3'}$ ≈ 10.2 Hz, $J_{3',4'}$ = 3.2 Hz, H-3'), 3.84 (s, COO$CH_3$), 2.58 (dd, $J_{3''a,3''e}$ = 12.6 Hz, $J_{3''eq,4''}$ ≈ 4.6 Hz, H-3"*eq*), 2.49 (t, $J_{vic}$ = 7.0 Hz, S$CH_2$-), 2.43 (t, $J_{vic}$ = 7.0 Hz, S$CH_2$-), 2.25-1.85 (each s, NHAc, OAc), 1.55 (br, OCH$_2$$CH_2$CH$_2$$CH_2$CH$_2$S), 1.39 (br, 6 H, -$CH_2$CH$_2$CH$_2$S), 0.92 (br, 6 H, Si$CH_2$).

I. R. data (KBr) : ν (cm$^{-1}$) 2941 (C-H), 1749 (C=O, ester), 1687 (C=O, amide), 1543 (N-H, amide), 1435 (C-N), 1230 (C-O, ester), 1039 (C-O, ether).

FAB-MS *Anal. Calc.* for $C_{162}H_{233}N_3O_{87}S_3Si$ $[M+Na]^+$ : 3761.2785. Found : $[M+Na]^+$ : 3760.84.

(2) Dumbbell-(1)6-2,3-SLac-Ac (compound x)

Dumbbell (1) 6-Br dendrimer (abbreviated) (compound iv: 12.8 mg, 0.01375 mmol) and compound viii (200 mg: 0.165 mmol) were dissolved in DMF (0.2 mL) until a homogeneous system was obtained. After dissolving, methanol (0.2 mL) was added followed by stirring at room temperature for 2 hours. Sodium methoxide (9.8 mg, 0.182 mmol) was added followed by stirring over night, and acetic acid (0.1 mL) was added thereto followed by stirring for several minutes and by subjecting to concentration. Following concentration, acetic anhydride (1 mL) and pyridine (1 mL) were added followed by stirring; after the completion of acetylation, the reaction solution was concentrated and extracted with cold 1 M hydrochloric acid, saturated saline solution and chloroform, consecutively, and the organic layer was dried over anhydrous sodium sulfate. The organic layer was filtrated, the filtrate was concentrated and dissolved in methanol and diethyl ether (1:1 (v/v)) and a solution of diazomethane in ether was added dropwise. Acetic acid was added to the reaction solution and the mixture was concentrated and purified by silica gel column chromatography [chloroform-methanol (30:1 v/v) silica gel 30 mL] to give dumbbell(1)6-2, 3-SLac-Ac (compound x: 79 mg, 76.7%).

Identification results are shown in Table 13.

Table 13

$R_f$ 0.3 [10:1 (v/v) chloroform-methanol];

N. M. R. data : $^1$H ($CDCl_3$), δ 7.26 $CHCl_3$, δ 5.50 (m, H-8"), 5.36 (dd, 3 H, $J_{6'',7''}$ = 2.7 Hz, $J_{7'',8''}$ = 9.1 Hz, H-7"), 5.19-4.59 (m), 4.49 (dd, 1 H, $J_{2',3'}$ = 10.4 Hz, $J_{3',4'}$ = 3.0 Hz, H-3'), 4.43-3.70 (m), 3.81 (s, $COOCH_3$), 3.62 (m, 12 H, H-5, H-6"), 3.43 (m, 6 H, one of $OCH_2$-), 2.55 (dd, H-3"eq), 2.46 (t, $J_{vic}$ = 7.0 Hz, $SCH_2$-), 2.45 (t, $J_{vic}$ = 7.0 Hz, $SCH_2$-), 2.43-1.83 (m, NH*Ac*, O*Ac*, -*CH*), 1.53 (br), 1.38 (br), 1.23 (br, $SiCH_2CH_2CH_2Si$), 0.56 (br, 20 H, $SiCH_2$-), -0.09 (s, 6 H, $SiMe_2$).

I. R. data (KBr) : ν ($cm^{-1}$) 2941 (C-H), 1749 (C=O, ester), 1670 (C=O, amide), 1541 (N-H, amide), 1437 (C-N), 1234 (C-O, ester), 1041 (C-O, ether).

FAB-MS *Anal.* Calc. for $C_{520}H_{474}N_6O_{174}S_6Si_3$ $[M+Na]^+$ : 7488.6080. Found : $[M+Na]^+$ : 7488.30.

(3) Fan-(O)3-2,3-SLac-OH (compound xi)

Fan(0)3-2, 3-SLac-AC dendrimer (abbreviated) (compound ix) was dissolved in methanol, and sodium methoxide (cat.) was added there to followed by over night stirring at room temperature. After the reaction solution was neutralized with a strongly acidic cation-exchange resin, IR-120B ($H^+$ type), the ion-exchange resin was collected by filtration and the filtrate was concentrated and subjected topurificationbygel filtration using Sephadex G-25 (5%AcOH) to give the deprotected fan (0) 3-2, 3-SLac-OH (compound xi), quantitatively as a freeze-dried powder.

Identification results are shown in Table 14.

Table 14

N. M. R. data : $^1H$ ($D_2O$), HDO δ 4.70, δ 7.30 (m, 5 H, Si*Ph*), 4.45 (br, 3 H, H-1'), 4.32 (br, 3 H, H-1), 4.07 (near d, 3 H, H-3'), 3.98-3.46 (m), 3.24 (br, 3 H, H-2), 2.66 (near d, 3 H, H-3"eq), 2.38 (br, 12 H, $-CH_2SCH_2-$), 1.95 (s, 9 H, NDAc), 1.84, 1.46, and 1.32 (3 m), 0.81 (br, 6 H, $CH_2Si$).

* δ 2.33 (near dd, H-3"eq of Lactone) Included about 8 %.

I. R. data (KBr) : ν ($cm^{-1}$) 3396 (O-H), 2927 (C-H), 1730 (C=O, carboxyl acid), 1633 (C=O, amide), 1566 (N-H, amide), 1072, and 1034 (C-O, alcohol, ether), 706, and 619 (N-H, out-of-plane bending)

FAB-MS *Anal.* Calc. for $C_{99}H_{167}N_3O_{57}S_3Si$ $[M+H]^+$ : 2433.9146. Found : 2434.1.

(4) Dumbbell (1) 6-2,3-SLac-OH (compound xii)

Dumbbell (1) 6-2,3-SLac-AC dendrimer (abbreviated) (compound x) (45.4 mg, 0.00608 mmol) was dissolved in methanol (3 mL), and sodium methoxide (1.97 mg, 0.03648 mmol) was added thereto followed by stirring at room temperature for one night. After the reaction solution was neutralized with a strongly acidic cation-exchange resin, IR-120B ($H^+$ type), the ion-exchange resin was collected by filtration and the filtrate was concentrated. To the residue was added a 0.05M aqueous solution of sodium hydroxide (2 mL) followed by stirring at room temperature over night. After the reaction solution was neutralized with a strongly acidic cation-exchange resin, IR-120 B ($H^+$ type), the ion-exchange resin was collected by filtration and the filtrate was concentrated and subjected to purification by gel filtration using Sephadex G-25 (5% AcOH) to give the deprotected dumbbell(1)6-Lac-OH (compound xii), quantitatively, as a freeze-dried powder.

Identification results are shown in Table 15.

Table 15

N. M. R. data : $^1$H ($D_2O$), HDO δ 4.70, δ 4.46 (near d, 6 H, H-1'), 4.36 (near d, 6 H, H-1), 4.08 (near d, 6 H, H-3'), 3.99-3.46 (m), 3.26 (br, 6 H, H-2), 2.67 (near d, 3 H, H-3"eq), 2.50 (br, 24 H. $-CH_2SCH_2-$), 1.96 (s, 18 H, NDAc), 1.85, 1.57, and 1.41 (3 br), 0.63 (br, 20 H, Si $CH_2$), -0.06 (br, 6 H, $SiMe_2$).

* δ 2.23 (near dd, $J_{3''a,3''e}$ = 12.9 Hz, $J_{3''a,4''}$ = 4.8 Hz, H-3"eq of Lactone) Included about 3%.

I. R. data (KBr) : ν ($cm^{-1}$) 3406 (O-H), 2931 (C-H), 1730 (C=O,carboxyl acid), 1643 (C=O, amide), 1566 (N-H, amide), 1032 (C-O, alcohol, ether), 624 (N-H, out-of-plane bending)

FAB-MS *Anal.* Calc. for $C_{194}H_{342}N_6O_{114}S_6Si_3$ $[M+H]^-$ : 4857.8769. Found : 4859.2.

<Example 3> Verotoxin neutralizing activity of the sugar chain-containing carbosilane dendrimer compounds

(1) Preparation

(a) Materials

The neutralizing activity of the sugar chain-containing carbosilane dendrimer compound synthesized in Example 1 as well as various dendrimer compounds including sugar chain-containing carbosilane dendrimers which were previously reported by the inventors against verotoxin were tested. All of the sugar chain-containing carbosilane dendrimer compounds used for the tests were synthesized by or in accordance with Example 1; their structures are as follows.

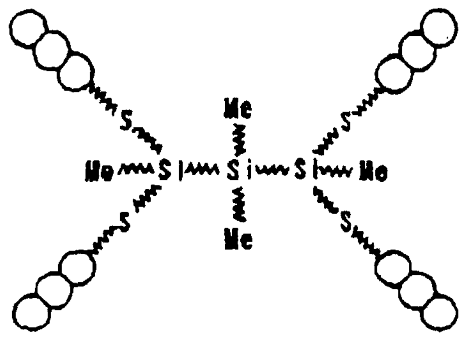

Dumbbell(1)4

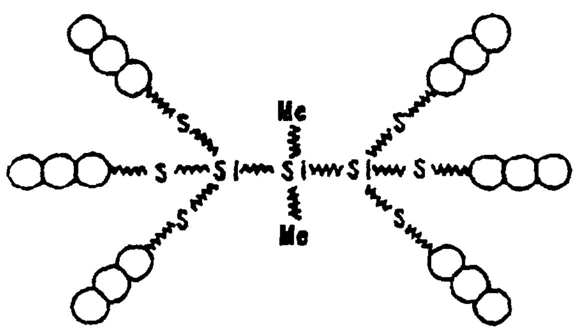

Dumbbell(1)6

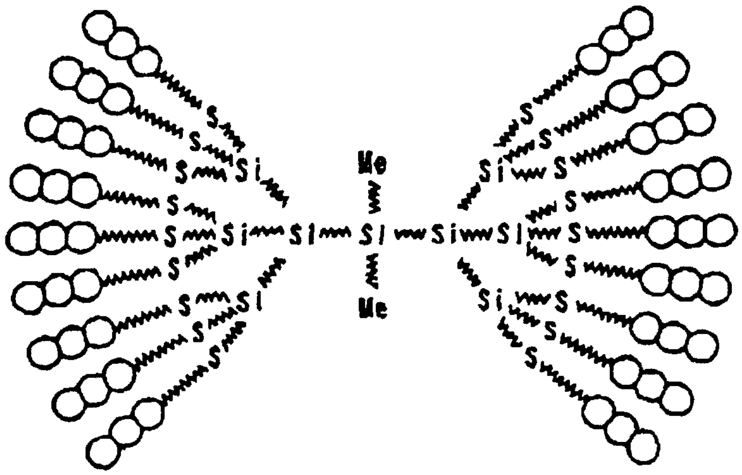

Dumbbell(2)18

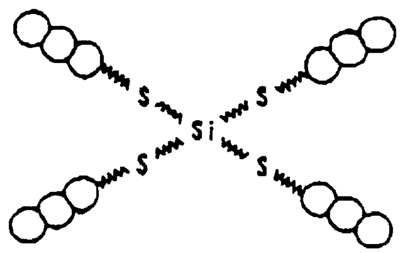

Ball(0)4

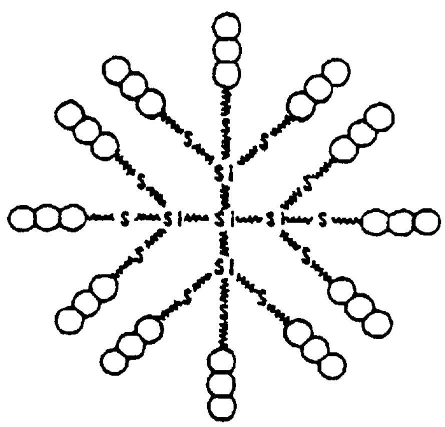

Ball(1)12

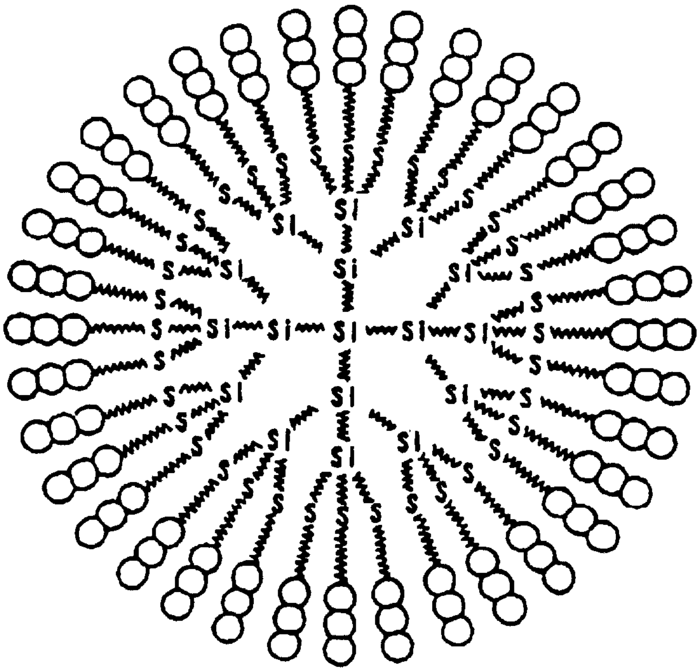

Ball(2)36

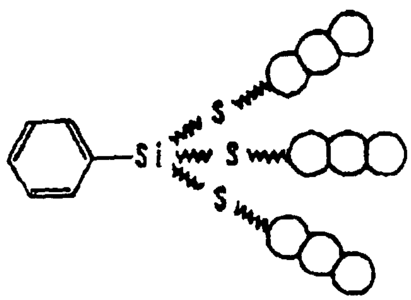

Fan(0)3

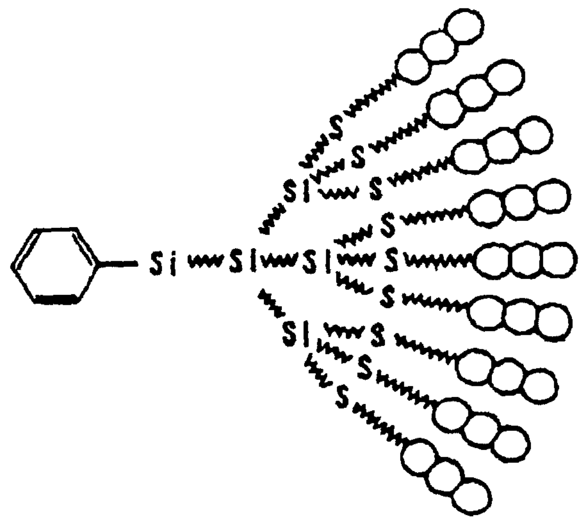

Fan(1)9

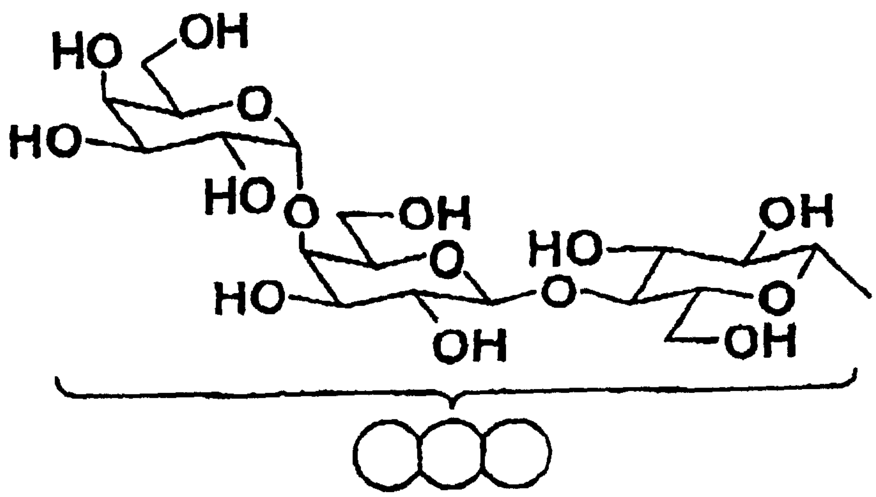

For the tests, verotoxins 1 and 2 (recombinants Stx1 and Stx2) were prepared according to known methods (such as *Microb. Pathog.*, 2, 339-349 (1987)).

Recombinant glutathione S-transferase (GST)-fused Stx1 (Stx1-A2$B_5$-GST) was obtained by the following method: *Bam*HI-EcoRI fragment was prepared by PCR using pUC118 vector having a sequence coding Stx1 (*Microb. Lett.* **44**, 23-26 (1987)) and using oligonucleotides of SEQ ID NO: 1 and SEQ ID NO: 2 as primers. The resulting fragment was bonded to a *Bam*HI-EcoRI site of pGEX-2T vector (Pharmacia Co.).

This Stx1-A2$B_5$-GST was expressed in bacteria and purified by known methods using glutathione-Sepharose beads (*J. Biol. Chem.*, **273,** 23126-23133 (1998)).

Further, hybridoma 13C4, which produces monoclonal antibody against the B subunit of Stx1, was obtained from the American Type Culture Collection.

Furthermore, $^{125}$I-labeled Stx1 ($^{125}$I-Stx1) and Stx2 were prepared by known iodine monochloride methods (*J. Biol. Chem.* **265,** 5226-5231 (1990)).

(b) Cells

Vero cells (cells derived from kidney of African green monkey) were cultured in a 24-well (for the testing binding property) or 96-well (for testing cytotoxicity) plastic microplate using Dulbecco's modified Eagle's medium (DMEM).

Peritoneal macrophage of mouse was prepared by known methods (*J. Biol. Chem.* **265,** 5226-5231 (1990)).

(c) Thin layer chromatography (TLC) immunostaining assay

Binding assay of Stx to Gb3 was performed according to known methods (*FEBS Lett*. **442,** 231-234 (1999)). Thus, porcine erythrocyte Gb3 (500 ng; Wako Pure Chemical) was applied to an HPTLC plate (Whatmanplc.) and developed using a mixed solvent of chloroform/methanol/water 60/35/8 (v/v); after blocking, it was incubated along with Stx1 (100 ng/ml) and an arbitrary amount of the sugar-containing carbosilane dendrimer. After washing, Stx1 was detected using monoclonal antibody 13C4.

(2) Methods

(a) Binding inhibition test

Vero cells were treated at 4°C for 30 minutes in the presence of $^{125}$I-Stx1 or $^{125}$I-Stx2 ($7 \times 10^6$ cpm/μg or $2 \times 10^8$ cpm/μg) and an arbitrary amount of the sugar-containing carbosilane dendrimer compound; after washing, the cells were dissolved in a dissolving solution (0.1 M NaOH, 0.5% SDS).

Radiation dose was measured by a γ-counter (Packard Inst. Comp.) , and the binding inhibition activity the sugar-containing carbosilane dendrimer compound was determined from the amount of $^{125}$I-Stx1 or $^{125}$I-Stx2 that bound to the vero cells.

(b) Cytotoxicity neutralization activity test

Stx1 or Stx2 (10 pg/ml) was added to sub-confluent vero cells in a 96-well plate in the presence of an arbitrary amount of the above-described sugar chain-containing carbosilane dendrimer compound and treated for 72 hours. The relative cell numbers were determined by conventional methods (*FEBS Lett.* **442,** 231-234 (1999) using a WST-1 cell counting kit (Wako Pure Chemical).

(c) Intravenous administration test in mice

A lethal dose of Stx1 or Stx2 (0.25 ng/g body weight) was administered from the tail vein of 5-10 ICRmice (body weight: 18-20 g; Nippon SLC) along with the various sugar chain-containing carbosilane dendrimers mentioned above and their surval time (days) was measured.

(3) Results

(a) Binfing inhibition activity

The 50% inhibition concentrations obtained from the binding inhibition activity test of the sugar chain-containing carbosilane dendrimer compounds are shown in Table 16.

Table 16 shows that fan(1)9 and the dumbbell type and a ball type sugar-containing carbosilane dendrimer compounds all show high binding inhibition activity against verotoxins 1 (Stx1) and 2 (Stx2).

When the sugar-containing carbosilane dendrimer compound was not present, 100% of the $^{125}$I-labeled Stx bound to the vero cells. Since the binding of $^{125}$I-labeled Stx to vero cells was completely inhibited even when the unlabeled Stx1 and Stx2 (50 μg/ml) were used instead of the sugar-containing carbosilane dendrimer compounds, it was confirmed that $^{125}$I-labeled Stx was bound to the vero cells, specifically.

(b) Cytotoxicity neutralization inhibition activity

The 50% suppressive concentration obtained from the toxicity neutralization inhibition activity test of various sugar-containing carbosilane dendrimers obtained in the same manner as in (a) is shown in Table 16.

Table 16 shows that all of the sugar chain-containing carbosilane dendrimers, particularly dumbbell (2) 18, show high cytotoxicity neutralizing activity against verotoxins 1 (Stx1) and 2 (Stx2).

Table 16

| Compounds | Binding Inhibition Activity $IC_{50}$ (µg/nL) | | Cytotoxin-Neutralization Activity $IC_{50}$ (µg/nL) | |
|---|---|---|---|---|
| | Stx1 | Stx2 | Stx1 | Stx2 |
| Fan (0) 3 | 43 | >100 | >100 | >100 |
| Fan (1) 9 | 0.32 | 6.1 | 32 | 13 |
| Dumbbell (1) 6 | 0.31 | 2.0 | 0.22 | 0.19 |
| Dumbbell (1) 4 | 0.42 | 0.85 | 0.24 | 0.40 |
| Dumbbell (2) 18 | 0.20 | 1.2 | 0.1 | 0.13 |
| Ball (1) 12 | 0.20 | 1.6 | 0.12 | 0.16 |
| Ball (2) 36 | 0.20 | 5.5 | 22.0 | 14 |

(c) Intravenous administration to mice

With regard to the above-mentioned dumbbell(1)6 and ball(1)12 compounds, the survival rates of mice when 50 µg/g body weight of the compounds alone were administered (○), when a lethal dose (0.25 ng/g body weight) of Stx 2 alone was administered (×) and when Stx2 (0.25 ng/g body weight) was administered together with each of the compounds (50 µg/g body weight) (●) are shown in Fig. 1 (A) and (B).

Fig. 1 shows that the ball(1)12 compound is effective to delay the death of mice caused by Stx2. Further, it is shown that the dumbbell (1) 12 compound can completely neutralize the cytotoxicity of Stx2.

Similarly, the survival rate of mice when a lethal dose (0.25 ng/g body weight) of Stx2 and each concentrations (1.5, 5, 15 and 50 µg/g body weight) of the dumbbell(1)12 compound were administered is shown in Fig. 2.

Fig. 2 shows that the survival rate varied among individual mice when the concentrations were 1.5 and 5 µg/g body weight, and the survival rate after 20 days was 30% . It was also confirmed that when 15 µg/g body weight of the dumbbell(1)12 compound were administered, the cytotoxicity of Stx2 was completely neutralized.

<Example 4> Anti-influenza virus activity of sugar-containing carbosilane dendrimer compounds

The erythrocytes aggregation inhibition activity, cell fusion inhibition activity and cell infection inhibition activity against influenza A virus (A/PR/8/34 strain) of the sialyl lactose derivative compound vii synthesized in Reference Example 4, the fan type compound xi and the dumbbell type compound xii synthesized in Examples 2(3) and 2(4) were tested using hepatic epithelial cells of dog (MDCK cells).

The results are shown in Table 17.

Table 17

| Compounds | Inhibiting Activity (µM) | | |
|---|---|---|---|
| | Erythrocyte Aggregation | Cell Fusion ($IC_{50}$) | Infection ($IC_{50}$) |
| vii | 133.3 | 745.3 | 124.2 |
| xi | 31.3 | 240.7 | 32.3 |
| xii | 15.6 | 31.2 | 5.4 |

From Table 17, particularly high activity against influenza virus was noted for compounds xi and xii, wherein sialyllactose is bound to carbosilane dendrimer.

Industrial Applicability

As described in detail above, a novel sugar chain-containing carbosilane dendrimer that show functions such as neutralization activity against verotoxin produced by *Escherichia coli* 0-157 is provided by the present invention.

Further, the present invention provides a novel carbosilane dendrimer containing sialyllactose at its terminus

as a substance that shows high antiviral property. In the carbosilane dendrimer compounds containing sialyllactose, molecular designing and synthesis of the carbosilane dendrimer moiety are relatively easy. In addition, such compounds are capable of binding and holding many sialyllactose molecules that show antiviral activity; further, their intermolecular space can be adjusted. Accordingly, they are highly useful as antiviral agents for the effective adhesion and removal of viruses.

## Claims

**1.** A sugar chain-containing carbosilane dendrimer represented by the following formula (I):

$$(R^1)_m-Si\left\{-R^2-Si[R^6]_l,\ [R^3-Si-(R^7)_k,\ (R^4-S-R^5-A)_{3-k}]_{3-l}\right\}_n \qquad (I)$$

(wherein, $R^1$, $R^6$ and $R^7$ may be the same or different and are hydrocarbon groups that may contain a substituent; $R^2$, $R^3$, $R^4$ and $R^5$ may be the same or different and are hydrocarbon chains that may contain a substituent; A is a sugar chain; m is a number selected from 0 to 3; n is a number selected from 4 to 1; m + n = 4; and k and 1 are the same or different numbers selected from 0 to 2).

**2.** A sugar chain-containing carbosilane dendrimer represented by the following formula (II):

$$(R^1)_m-Si\left[-R^2-Si(R^6)_l\ (R^4-S-R^5-A)_{3-l}\right]_n \qquad (II)$$

(wherein, $R^1$ and $R^6$ are hydrocarbon groups that may contain a substituent; $R^2$, $R^4$ and $R^5$ may be the same or different and are hydrocarbon groups that may contain a substituent; A is a sugar chain; m is a number selected from 0 to 3; n is a number selected from 4 to 1; m + n = 4; and 1 is a number selected from 0 to 2).

**3.** The sugar chain-containing carbosilane dendrimer of claims 1 or 2, wherein $R^1$ is a phenyl group, m is 1 and n is 3.

**4.** The sugar chain-containing carbosilane dendrimer of claims 1, 2 or 3, wherein $R^1$ is a methyl group and m and n are both 2.

**5.** The sugar chain-containing carbosilane dendrimer of claims 1, 2, 3 or 4, wherein the sugar chain A is represented by the following formula (III):

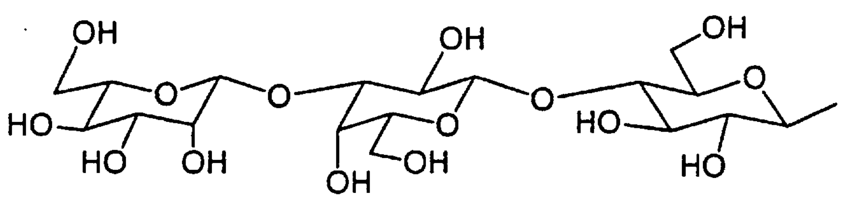

(III)

**6.** The sugar chain-containing carbosilane dendrimer of claims 1, 2, 3 or 4, wherein the sugar chain A is represented by the following formula (IV):

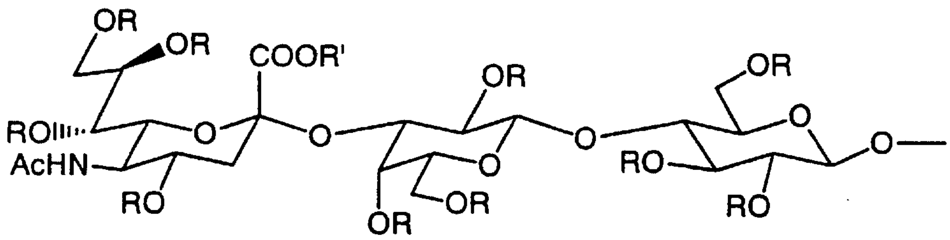

(IV)

(wherein, R is a hydrogen atom or an acetyl group and $R^1$ is a hydrogen atom or a methyl group).

7. A method for producing the sugar chain-containing carbosilane dendrimer of claim 1, comprising reacting a halogenated compound represented by the following formula (V):

$$(R^1)_m-Si\left\{-R^2-Si\,[R^6]_l\,,\quad [\,R^3-Si-(R^7)k\,,\quad (R^4-X)_{3-k}\,]_{3-l}\right\}_n \qquad (V)$$

(wherein, $R^1$, $R^2$, $R^3$, $R^4$, $R^6$, $R^7$, m, n, k and l are as defined above and X is halogen atom) with a sulfide compound represented by the following formula (VI):

$$A-R^5-S-Y \qquad (VI)$$

(wherein, $R^5$ and A are as defined above and Y is a protective group that is released upon reaction).

8. A method for producing the sugar chain-containing carbosilane dendrimer of claim 2, comprising reacting a halogenated compound represented by the following formula (VII):

$$(R^1)_m-Si\left[-R^2-Si\,(R^6)_l\quad (R^4-X)_{3-l}\right]_n \qquad (VII)$$

(wherein, $R^1$, $R^2$, $R^4$, $R^6$, m, n and l are as defined above and X is a halogen atom) with a sulfide compound represented by the following formula (VI):

$$A-R^5-S-Y \qquad (VI)$$

(wherein, $R^5$ and A are as defined above and Y is a protective group that is released upon reaction).

9. The method for producing sugar chain-containing carbosilane dendrimer of claim 7 or 8, wherein A in formula (VI) is represented by the following formula (III):

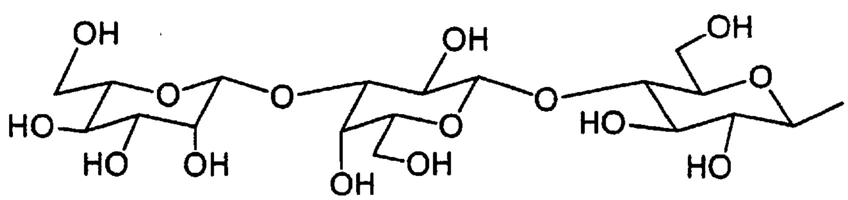

(III)

10. The method for producing sugar chain-containing carbosilane dendrimer of claim 7 or 8, wherein A in formula (VI) is represented by the following formula (IV):

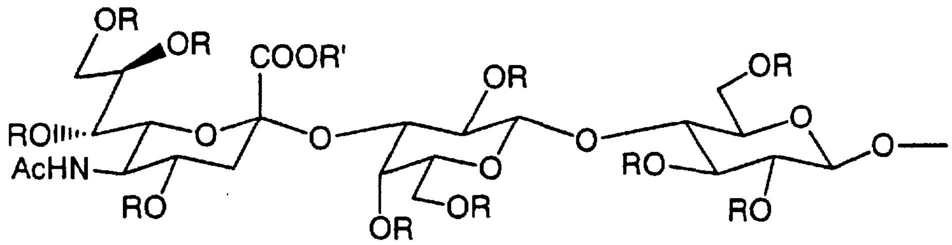

(IV)

11. The method for producing sugar chain-containing carbosilane dendrimer of claim 9 or 10, wherein Y in formula (VI) is an acetyl group is provided.

12. The method for producing sugar chain-containing carbosilane dendrimer of claim 10 or 11, wherein a thiomethyl glucoside compound of N-acetylneuraminic acid and a trimethylsilyl ethyl glycoside 3',4'-diol compound of lactose are subjected to glycosidation to produce the sialyl lactose derivative of the following formula (VIII):

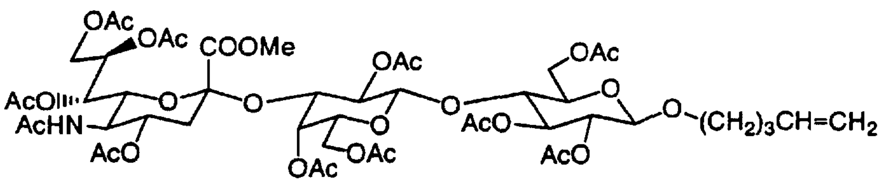

(VIII)

to which thioacetic acid is added by radical addition reaction, thereby producing the acetylthio compound represented by the following formula (IX)

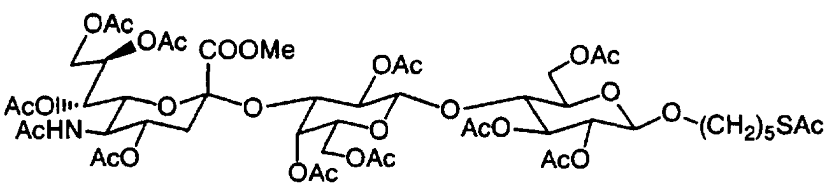

(IX)

to which the carbosilane dendrimer of formula (V) or (VII) are condensated.

13. A neutralizing agent for verotoxin, comprising the sugar chain-containing carbosilane dendrimer of claims 1, 2, 3, 4, 5 or 6 as an effective ingredient.

14. An antiviral agent comprising the sugar chain-containing carbosilane dendrimer of claims 1, 2, 3, 4, 5 or 6 as an effective ingredient.

*Fig. 1*

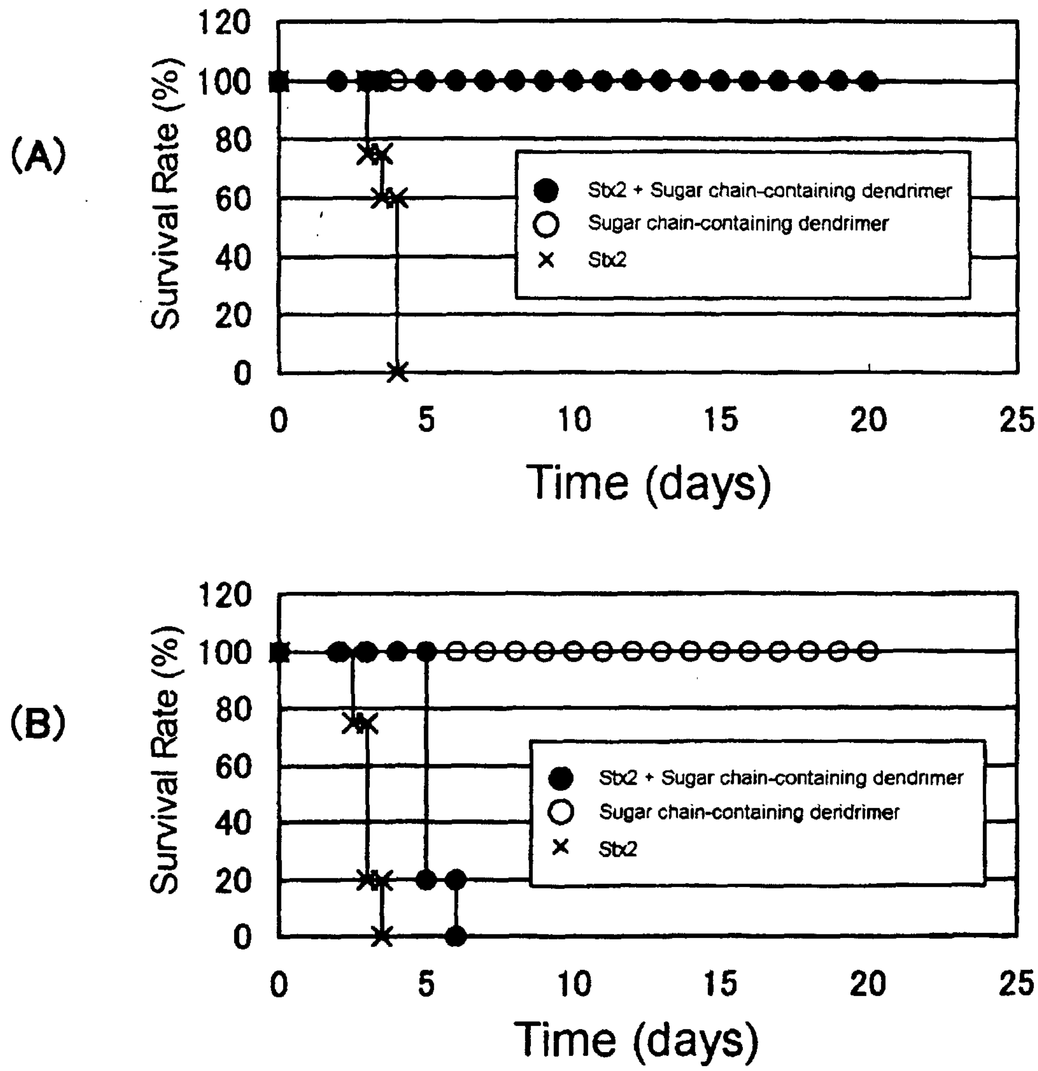
(A)
Survival Rate (%)
120
100
80
60
40
20
0
0
5
10
15
20
25
Time (days)
Stx2 + Sugar chain-containing dendrimer
Sugar chain-containing dendrimer
Stx2
(B)
Survival Rate (%)
120
100
80
60
40
20
0
0
5
10
15
20
25
Time (days)
Stx2 + Sugar chain-containing dendrimer
Sugar chain-containing dendrimer
Stx2

*Fig. 2*

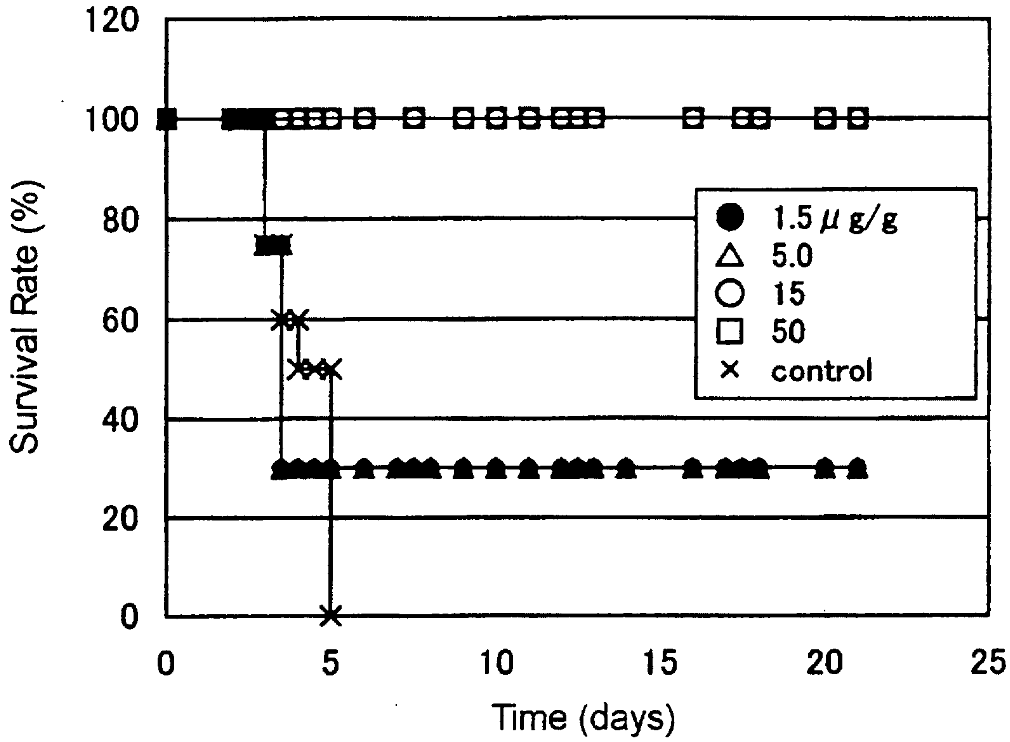
120
100
80
60
40
20
0
Survival Rate (%)
0
5
10
15
20
25
Time (days)
1.5 μ g/g
5.0
15
50
control

## INTERNATIONAL SEARCH REPORT

International application No.
PCT/JP01/05717

A. CLASSIFICATION OF SUBJECT MATTER
Int.Cl$^7$ C07H23/00, A61K31/702, A61P31/04, 12

According to International Patent Classification (IPC) or to both national classification and IPC

B. FIELDS SEARCHED

Minimum documentation searched (classification system followed by classification symbols)
Int.Cl$^7$ C07H23/00, A61K31/70-714

Documentation searched other than minimum documentation to the extent that such documents are included in the fields searched

Electronic data base consulted during the international search (name of data base and, where practicable, search terms used)
REGISTRY (STN), CAPLUS (STN), CA (STN), CAOLD (STN), WPI/L (DIALOG), JOIS (JICST)

C. DOCUMENTS CONSIDERED TO BE RELEVANT

| Category* | Citation of document, with indication, where appropriate, of the relevant passages | Relevant to claim No. |
|---|---|---|
| X<br>Y | Koji MATSUOKA, et al., "Synthetic assembly of trisaccharide moieties of globotriaosyl ceramide using carbosilane dendrimers as cores. A new type of functional glyco-material", Tetrahedron Letters, (1999), Vol.40, pages 7839 to 7842<br>Full text | 1-5,7-9,11, 13<br>6,10,12,14 |
| Y | JP 11-147951 A (Rikagaku Kenkyusho), 02 June, 1999 (02.06.99), (Family: none)<br>Full text | 6,10,12,14 |
| PX | Koji MATSUOKA, et al., "An alternative route for the construction of carbosilane dendrimers uniformly functionalized with lactose or siallyllactose moieties", Tetrahedron Letters, 07 May, 2001, Vol.42, pages 3327 to 3330<br>Full text | 1-4,6-8, 10-12 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search<br>03 August, 2001 (03.08.01) | Date of mailing of the international search report<br>14 August, 2001 (14.08.01) |
|---|---|
| Name and mailing address of the ISA/<br>Japanese Patent Office<br><br>Facsimile No. | Authorized officer<br><br>Telephone No. |

Form PCT/ISA/210 (second sheet) (July 1992)